# EUROPEAN PATENT APPLICATION

(11) **EP 4 607 522 A1**
(43) Date of publication of application: **27.08.2025**
(21) Application number: 23879714.6
(22) Date of filing: 12.10.2023
(51) Int. Cl.: G16C 60/00, G06N 3/02

(54) **COMPOSITION OPTIMIZATION DEVICE, COMPOSITION OPTIMIZATION METHOD, AND COMPOSITION OPTIMIZATION PROGRAM**

(30) Priority: 17.10.2022 JP 2022166274
(71) Applicant: Resonac Corporation, Tokyo 105-7325 (JP)
(72) Inventor: OKANO, Yu, Tokyo 105-7325 (JP); KANESHITA, Takeshi, Tokyo 105-7325 (JP); TAKEMOTO, Shimpei, Tokyo 105-7325 (JP)
(74) Representative: Strehl & Partner mbB
(86) International application number: PCT/JP2023/037117
(87) International publication number: WO 2024/085071

(57) **Abstract**

A material composition corresponding to a target phase fraction is efficiently searched for. A composition optimization device includes a prediction unit configured to predict, by inputting a predetermined material composition into a trained model, a phase fraction of a material having the predetermined material composition at each temperature within a predetermined temperature range, the trained model being trained using training data in which a material composition of a training target material and a phase fraction of the training target material at each temperature within the predetermined temperature range are associated with each other; and an update unit configured to update the predetermined material composition input into the trained model by performing backpropagation of an error calculated based on a target phase fraction and the predicted phase fraction. A process of the prediction unit predicting a phase fraction based on the updated material composition and a process of the update unit updating a predetermined material composition by performing backpropagation of a calculated error are repeated until a calculated error satisfies a predetermined condition.

## Description

### TECHNICAL FIELD

The present disclosure relates to a composition optimization device, a composition optimization method, and a composition optimization program.

### BACKGROUND

In designing a material such as an alloy, it is important to calculate a phase fraction in a thermodynamic equilibrium state over a predetermined temperature range. As an example, a model for predicting a phase fraction from a material composition is known. By using the model, for example, it is possible to predict how a phase fraction changes when a material composition is changed.

### Related Art Document

### Patent Document

[Patent Document 1] WO 2020/090617

### SUMMARY OF THE INVENTION

### Problem to be solved by the invention

With respect to the above, in the above model, the number of model parameters is great, and thus it takes a certain amount of time to predict the phase fraction. Therefore, if attempting to solve an enormous number of forward problems while exhaustively changing a material composition in order to search for a material composition corresponding to a target phase fraction (that is, in order to solve an inverse problem), an appropriate material composition cannot be searched for within a realistic time.

The present disclosure aims to efficiently search for a material composition corresponding to a target phase fraction.

### Means for Solving the Problem

A composition optimization device according to a first aspect includes:
a prediction unit configured to predict, by inputting a predetermined material composition into a trained model, a phase fraction of a material having the predetermined material composition at each temperature within a predetermined temperature range, the trained model being trained using training data in which a material composition of a training target material and a phase fraction of the training target material at each temperature within the predetermined temperature range are associated with each other; and
an update unit configured to update the predetermined material composition input into the trained model by performing backpropagation of an error calculated based on a target phase fraction and the predicted phase fraction,
wherein a process of the prediction unit predicting a phase fraction based on the updated material composition and a process of the update unit updating a predetermined material composition by performing backpropagation of a calculated error are repeated until the calculated error satisfies a predetermined condition.

A second aspect of the present disclosure is the composition optimization device described in the first aspect, wherein the trained model is configured to predict the phase fraction at an (i+1)-th temperature by using phase fractions predicted by the trained model with respect to temperatures up to an i-th temperature (i is an integer of 1 or greater) within the predetermined temperature range.

A third aspect of the present disclosure is the composition optimization device described in the second aspect, wherein an architecture configured to calculate time series data is applied to the trained model, and the trained model predicts the phase fraction for each predetermined temperature interval based on the predetermined material composition, the time series data being data for each predetermined time interval.

A fourth aspect of the present disclosure is the composition optimization device described in the third aspect, wherein the trained model is any one of an RNN, a bidirectional RNN, Seq2Seq, Seq2Seq with an attention mechanism, a GRU, an LSTM, or Transformer.

A fifth aspect of the present disclosure is the composition optimization device described in the fourth aspect, wherein the trained model includes:
an encoder configured to output a feature by the predetermined material composition being input; and
a decoder configured to predict the phase fraction at the (i+1)-th temperature by the output feature and the predicted phase fractions at the temperatures up to the i-th temperature being input.

A sixth aspect of the present disclosure is the composition optimization device described in any one of the first to fifth aspects, wherein the phase fraction is the phase fraction in a thermodynamic equilibrium state.

A seventh aspect of the present disclosure is the composition optimization device described in any one of the first to sixth aspects, further comprising a constraint unit configured to correct the updated predetermined material composition so that the updated predetermined material composition that is updated by the update unit satisfies a constraint related to a material composition.

An eighth aspect of the present disclosure is the composition optimization device described in the seventh aspect, wherein the constraint unit corrects the updated predetermined material composition so that a total value of the material composition is 100% and a ratio of each composition is 0% or greater.

A ninth aspect of the present disclosure is the composition optimization device described in any one of the first to eighth aspects, further comprising a loss function calculation unit configured to calculate the error calculated based on the target phase fraction and the predicted phase fraction.

A tenth aspect of the present disclosure provides the composition optimization device described in the ninth aspect, wherein the error calculated by the loss function calculation unit includes at least:
a first addition result obtained by adding, for all phases, the error between the predicted phase fraction and the target phase fraction at a temperature at which each phase specified based on the target phase fraction appears or disappears;
a second addition result obtained by adding the error between the predicted phase fraction and the target phase fraction at each temperature for the predetermined temperature range;
a third addition result obtained by adding an error between a logarithmic value of the predicted phase fraction and a logarithmic value of the target phase fraction at each temperature for the predetermined temperature range;
a fourth addition result obtained by adding an error between a difference value between phase fractions at adjacent temperatures among the predicted phase fraction at each temperature and a difference value between phase fractions at adjacent temperatures among the predicted phase fraction at each temperature for the predetermined temperature range, or
a fifth addition result obtained by adding an error between a ratio of the predicted phase fraction and a ratio of the target phase fraction at each temperature for the predetermined temperature range.

An eleventh aspect of the present disclosure is the composition optimization device described in the tenth aspect, wherein the loss function calculation unit performs weighted addition on the first addition result to the fifth addition result.

A twelfth aspect of the present disclosure is the composition optimization device described in the tenth aspect, wherein the loss function calculation unit makes the logarithmic value of the phase fraction non-negative by adding a value corresponding to a first decimal place of the phase fraction when calculating the logarithmic value of the phase fraction.

A thirteenth aspect of the present disclosure is the composition optimization device described in any one of the first to twelfth aspects, wherein the predetermined material composition is a material composition having a phase fraction similar to the target phase fraction selected from material compositions defined by a standard.

A fourteenth aspect of the present disclosure is a composition optimization method performed by a computer of a composition optimization device, comprising:
a prediction step of predicting, by inputting a predetermined material composition into a trained model, a phase fraction of a material having the predetermined material composition at each temperature within a predetermined temperature range, the trained model being trained using training data in which a material composition of a training target material and a phase fraction of the training target material at each temperature within the predetermined temperature range are associated with each other; and
an update step of updating the predetermined material composition input into the trained model by performing backpropagation of an error calculated based on a target phase fraction and the predicted phase fraction,
wherein a process of predicting a phase fraction based on the updated material composition in the prediction step and a process of updating a predetermined material composition by performing backpropagation of a calculated error in the update step are repeated until a calculated error satisfies a predetermined condition.

A fifteenth aspect of the present disclosure is a composition optimization program for causing a computer of a composition optimization device to perform steps comprising:
a prediction step of predicting, by inputting a predetermined material composition into a trained model, a phase fraction of a material having the predetermined material composition at each temperature within a predetermined temperature range, the trained model being trained using training data in which a material composition of a training target material and a phase fraction of the training target material at each temperature within the predetermined temperature range are associated with each other; and
an update step of updating the predetermined material composition input into the trained model by performing backpropagation of an error calculated based on a target phase fraction and the predicted phase fraction,
wherein a process of predicting a phase fraction based on the updated material composition in the prediction step and a process of updating a predetermined material composition by performing backpropagation of a calculated error in the update step are repeated until a calculated error satisfies a predetermined condition.

### Effect of the invention

According to the present disclosure, a material composition corresponding to a target phase fraction can be efficiently searched for.

### BRIEF DESCRIPTION OF THE DRAWINGS

[FIG. 1] FIG. 1 is a diagram illustrating an example of a system configuration of a material development support system and examples of functional configurations of a training device, a prediction device, and a composition optimization device.
[FIG. 2] FIG. 2 is a diagram illustrating an example of a hardware configuration of a training device, a prediction device, and a composition optimization device.
[FIG. 3] FIG. 3 is a flowchart illustrating a flow of a material development support process.
[FIG. 4] FIG. 4 is a diagram illustrating a configuration of training data and specific examples of input data and ground truth data.
[FIG. 5] FIG. 5 is a diagram illustrating an example of a functional configuration of a training data generation unit.
[FIG. 6] FIG. 6 is a diagram illustrating an example of a functional configuration of a training unit.
[FIG. 7] FIG. 7 is a first diagram illustrating an operation example of the training unit.
[FIG. 8] FIG. 8 is a second diagram illustrating an operation example of the training unit.
[FIG. 9] FIG. 9 is a diagram illustrating an example of a method of calculating a loss by a loss function calculation unit.
[FIG. 10] FIG. 10 is a flowchart illustrating a flow of a training process.
[FIG. 11] FIG. 11 is a diagram illustrating an example of a functional configuration of a prediction unit.
[FIG. 12] FIG. 12 is a diagram illustrating an operation example of a prediction unit.
[FIG. 13] FIG. 13 is a flowchart illustrating a flow of a prediction process.
[FIG. 14] FIG. 14 depicts graphs for explaining the prediction accuracy.
[FIG. 15] FIG. 15 is a first diagram illustrating an operation example of a forward propagation process in the composition optimization device.
[FIG. 16] FIG. 16 is a second diagram illustrating an operation example of the forward propagation process in the composition optimization device.
[FIG. 17] FIG. 17 is a third diagram illustrating an operation example of the forward propagation process in the composition optimization device.
[FIG. 18] FIG. 18 is a diagram illustrating an operation example of a backpropagation process in the composition optimization device.
[FIG. 19] FIG. 19 is a fourth diagram illustrating an operation example of the forward propagation process in the composition optimization device.
[FIG. 20] FIG. 20 is a flowchart illustrating a flow of a composition optimization process.
[FIG. 21] FIG. 21 is a flowchart illustrating a flow of a material composition update process.
[FIG. 22] FIG. 22 depicts first graphs indicating examples of a processing result of the composition optimization process.
[FIG. 23] FIG. 23 is a first diagram illustrating another example of the functional configuration of the composition optimization device.
[FIG. 24] FIG. 24 is a second diagram illustrating another example of the functional configuration of the composition optimization device.
[FIG. 25] FIG. 25 is a chart indicating an example of default material composition information.
[FIG. 26] FIG. 26 depicts charts indicating examples of the default material composition information and a processing result of the composition optimization process.
[FIG. 27] FIG. 27 depicts second graphs indicating examples of the processing result of the composition optimization process.

### DESCRIPTION OF THE EMBODIMENTS

In the following, each embodiment will be described with reference to the accompanying drawings. Here, in the present specification and drawings, components having substantially the same functional configuration are denoted by the same reference symbols, and duplicated description thereof will be omitted.

### [First Embodiment]

### <System Configuration of Material Development Support System, Functional Configurations of Training Device, Prediction Device, and Composition Optimization Device>

First, a system configuration of a material development support system including a training device, a prediction device, and a composition optimization device according to a first embodiment, and functional configurations of the training device, the prediction device, and the composition optimization device will be described. In the present embodiment, the material development support system is a system configured to:
- predict a phase fraction (a phase fraction in a thermodynamic equilibrium state, hereinafter, simply referred to as the "phase fraction") at each temperature within a predetermined temperature range by using a trained prediction model based on material composition information (analyze a forward problem); and
- search for material composition information corresponding to a target phase fraction at each temperature within a predetermined temperature range by using a trained prediction model (analyze an inverse problem).

In the material development support system according to the present embodiment, the phase fraction is provided as a graph illustrating a ratio of phase deposition of each composition at each temperature over a predetermined temperature range, for example. In this case, for a material such as an aluminum alloy, the material strength can be determined based on a shape of a graph of the phase fraction. Here, the phase fraction may be provided as a numerical value at each temperature, which is a ratio of phase deposition of each composition.

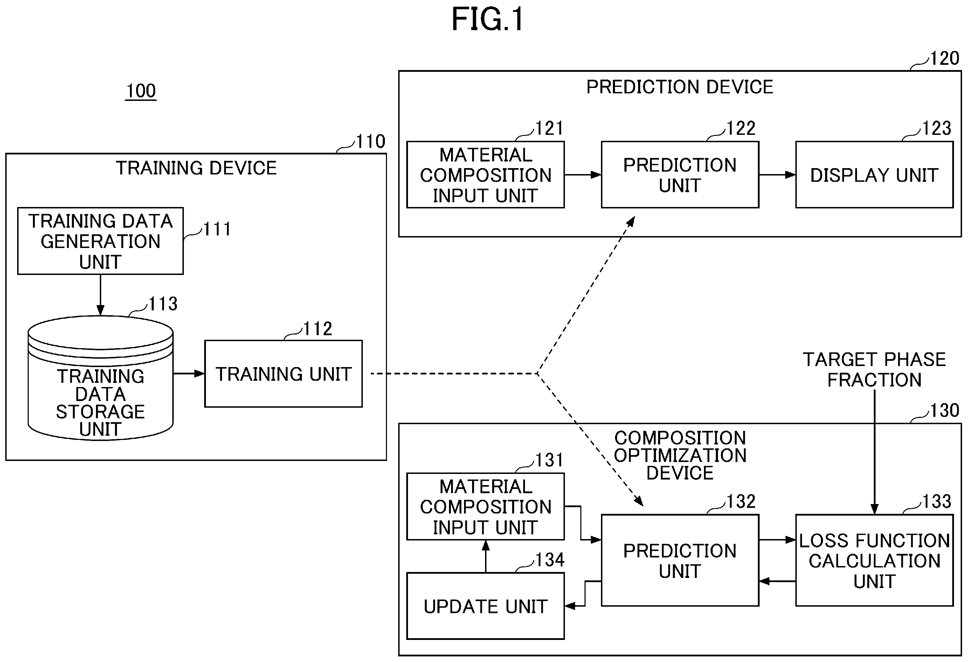

FIG. 1 is a diagram illustrating an example of the system configuration of the material development support system and an example of the functional configurations of the training device, the prediction device, and the composition optimization device. As illustrated in FIG. 1, a material development support system 100 includes a training device 110, a prediction device 120, and a composition optimization device 130.

A training program is installed in the training device 110, and by executing the program, the training device 110 functions as a training data generation unit 111 and a training unit 112.

The training data generation unit 111 generates training data for training a prediction model, and stores the training data in a training data storage unit 113. In the present embodiment, as the training data for training the prediction model, multiple pieces of material composition information and a phase fraction at each temperature within a predetermined temperature range for each of the multiple pieces of material composition information are stored in the training data storage unit 113 in association with each other.

The training unit 112 reads the training data from the training data storage unit 113 and trains the prediction model by using the read training data. The training unit 112 trains the prediction model so that output data in a case where the material composition information stored in the training data is input into the prediction model approaches the phase fraction at each temperature within the predetermined temperature range that is stored in association in the training data. With this, the training unit 112 generates a trained prediction model. Additionally, the training unit 112 stores the generated trained prediction model in the prediction unit 122 of the prediction device 120 and the prediction unit 132 of the composition optimization device 130.

Here, the training unit 112 applies, as a prediction model, an architecture (generally an architecture used for natural language processing or the like) capable of calculating time-series data, which is data for each predetermined time interval, that is, Seq2Seq with an attention mechanism; or Transformer. Thus, according to the prediction model, inputting the material composition information stored in the training data enables sequential outputting of data corresponding to the phase fraction at each temperature within the predetermined temperature range.

Here, "sequentially outputting output data corresponding to the phase fraction at each temperature" indicates that, for example, the prediction model outputs output data corresponding to the phase fraction at the (i+1)-th temperature by using ground truth data stored in the training data for the temperatures up to the i-th temperature. Here, i is an integer of 1 or greater.

As described, the training unit 112 is configured to sequentially output the output data corresponding to the phase fraction at each temperature (configured to process the output data corresponding to the phase fraction at each temperature as time-series data). With this, according to the training unit 112, training in which the output data corresponding to the phase fractions in the adjacent temperature regions is reflected can be performed.

A prediction program is installed in the prediction device 120, and by executing the program, the prediction device 120 functions as a material composition input unit 121, a prediction unit 122, and a display unit 123.

When material composition information on a prediction target material is input, the material composition input unit 121 receives the material composition information and notifies the prediction unit 122 of the material composition information.

The prediction unit 122 includes the trained prediction model trained by the training unit 112, and sequentially predicts the phase fraction at each temperature within the predetermined temperature range by inputting the material composition information notified from the material composition input unit 121 into the trained prediction model. That is, the prediction unit 122 sequentially predicts the phase fraction for each predetermined temperature interval within the predetermined temperature range.

Here, "sequentially predict the phase fraction for each predetermined temperature interval" indicates that, for example, by using the phase fractions predicted by the trained prediction model for the temperatures up to the i-th temperature (i is an integer of 1 or greater), the trained prediction model predicts the phase fraction at the (i+1)-th temperature.

As described, the prediction unit 122 is configured to sequentially predict the phase fraction at each temperature (that is, configured to process the phase fraction at each temperature as time-series data by a recursive network). With this, according to the prediction unit 122, prediction in which prediction data of the phase fractions in the adjacent temperature regions is reflected can be performed. As a result, for example, in comparison with a case where a multilayer neural network is applied (a case where the prediction results of the phase fractions in the adjacent temperature regions are not reflected), a decrease in the prediction accuracy of the phase fraction can be suppressed. That is, according to the prediction unit 122, the prediction accuracy can be improved in the trained prediction model configured to predict the phase fractions over the predetermined temperature range based on the material composition.

The display unit 123 displays the phase fraction at each temperature within the predetermined temperature range that is predicted by the prediction unit 122. The display unit 123 displays the phase fraction at each temperature within the predetermined temperature range that is predicted by the prediction unit 122, for example, in different colors for phases. Here, the display of the phase fraction as a graph may be performed by changing the type of line such as a dotted line or a chain line.

A composition optimization program is installed in the composition optimization device 130, and by executing the program, the composition optimization device 130 functions as a material composition input unit 131, a prediction unit 132, a loss function calculation unit 133, and an update unit 134.

When default material composition information is input, the material composition input unit 131 receives the default material composition information and notifies the prediction unit 132 of the default material composition information. Additionally, when updated material composition information that is updated by the update unit 134 is input, the material composition input unit 131 receives the updated material composition information and notifies the prediction unit 132 of the updated material composition information.

The prediction unit 132 includes the trained prediction model trained by the training unit 112, and sequentially predicts, by inputting the material composition information notified from the material composition input unit 131 into the trained prediction model, the phase fraction at each temperature within the predetermined temperature range. That is, the prediction unit 132 sequentially predicts the phase fraction for each predetermined temperature interval within the predetermined temperature range.

The loss function calculation unit 133 calculates multiple types of losses (errors) based on the phase fraction at each temperature within the predetermined temperature range that is sequentially predicted by the prediction unit 132 and the target phase fraction at each temperature within the predetermined temperature range that is set in advance. Additionally, the loss function calculation unit 133 calculates an integration loss by integrating the calculated multiple types of losses, and performs backpropagation of the calculated integration loss in a state where model parameters of the trained prediction model of the prediction unit 132 are fixed.

The update unit 134 updates the material composition information based on the backpropagation result output from the trained prediction model of the prediction unit 132 by the integration loss being propagated backward by the loss function calculation unit 133. Additionally, the update unit 134 inputs the updated material composition information into the material composition input unit 131.

As described, in the composition optimization device 130, when a material composition corresponding to the target phase fraction is searched for, the material composition is changed by backpropagation of the integration loss instead of exhaustively changing the material composition. With this, according to the composition optimization device 130, the material composition corresponding to the target phase fraction can be efficiently searched for.

### <Hardware Configurations of Training device, Prediction Device, and Composition Optimization Device>

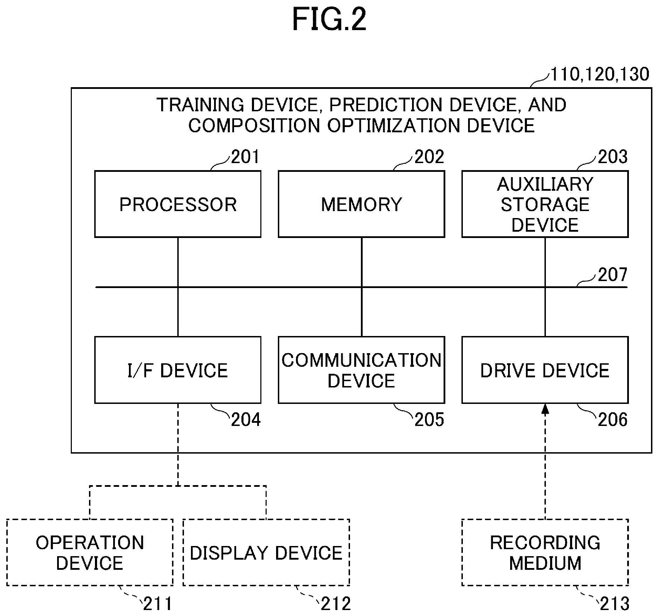

Next, hardware configurations of the training device 110, the prediction device 120, and the composition optimization device 130 will be described. Here, the training device 110, the prediction device 120, and the composition optimization device 130 have the same hardware configuration, and thus the hardware configurations of the training device 110, the prediction device 120, and the composition optimization device 130 will be collectively described here with reference to FIG. 2.

FIG. 2 is a diagram illustrating an example of the hardware configuration of the training device, the prediction device, and the composition optimization device. As illustrated in FIG. 2, the training device 110, the prediction device 120, and the composition optimization device 130 include a processor 201, a memory 202, an auxiliary storage device 203, an interface (I/F) device 204, a communication device 205, and a drive device 206. Here, the hardware components of each of the training device 110, the prediction device 120, and the composition optimization device 130 are connected to each other via a bus 207.

The processor 201 includes various computing devices, such as a central processing unit (CPU) and a graphics processing unit (GPU). The processor 201 reads various programs (for example, a training program, a prediction program, a composition optimization program, and the like) onto the memory 202 and executes the programs.

The memory 202 includes a main storage device, such as a read only memory (ROM) or a random access memory (RAM). The processor 201 and the memory 202 form what is called a computer, and the processor 201 executes various programs read on the memory 202, and thereby the computer realizes various functions.

The auxiliary storage device 203 stores various programs and various data used when the various programs are executed by the processor 201. For example, the training data storage unit 113 is implemented in the auxiliary storage device 203.

The I/F device 204 is a connection device that connects to an operation device 211 and a display device 212, which are examples of a user interface device. The communication device 205 is a communication device for communicating with an external device (not illustrated) via a network.

The drive device 206 is a device for setting a recording medium 213. The recording medium 213 herein includes a medium for optically, electrically, or magnetically recording information, such as a CD-ROM, a flexible disk, or a magnetooptical disk. Additionally, the recording medium 213 may include a semiconductor memory or the like that electrically records information, such as a ROM or a flash memory.

Here, the various programs to be installed in the auxiliary storage device 203 are installed by, for example, the distributed recording medium 213 being set in the drive device 206 and the various programs recorded in the recording medium 213 being read by the drive device 206. Alternatively, the various programs to be installed in the auxiliary storage device 203 may be installed by being downloaded from a network via the communication device 205.

### <Flow of Material Development Support Process>

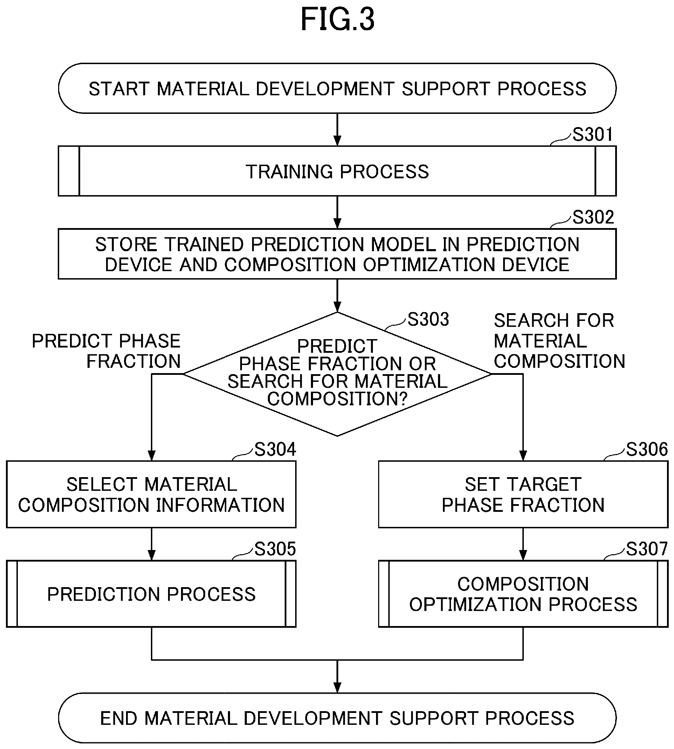

Next, a flow of an entire material development support process by the material development support system 100 will be described. FIG. 3 is a flowchart illustrating the flow of the material development support process.

In step S301, the training device 110 performs a training process and generates the trained prediction model.

In step S302, the prediction device 120 and the composition optimization device 130 store the trained prediction model.

In step S303, the prediction device 120 and the composition optimization device 130 determine whether to execute a task of predicting a phase fraction or a task of searching for a material composition.

When it is determined in step S303 that the task of predicting the phase fraction is to be executed, the process proceeds to step S304, and the prediction device 120 receives selection of the material composition information. Additionally, in step S305, the prediction device 120 executes a prediction process for the material composition information that has been received as the selection.

When it is determined in step S303 that the task of searching for the material composition is to be executed, the process proceeds to step S306, and the composition optimization device 130 receives a setting of the target phase fraction. Additionally, in step S307, the composition optimization device 130 performs a composition optimization process of searching for a material composition that achieves the target phase fraction that has been received as the setting.

### <Configuration of Training Data, Input Data, and Ground Truth Data>

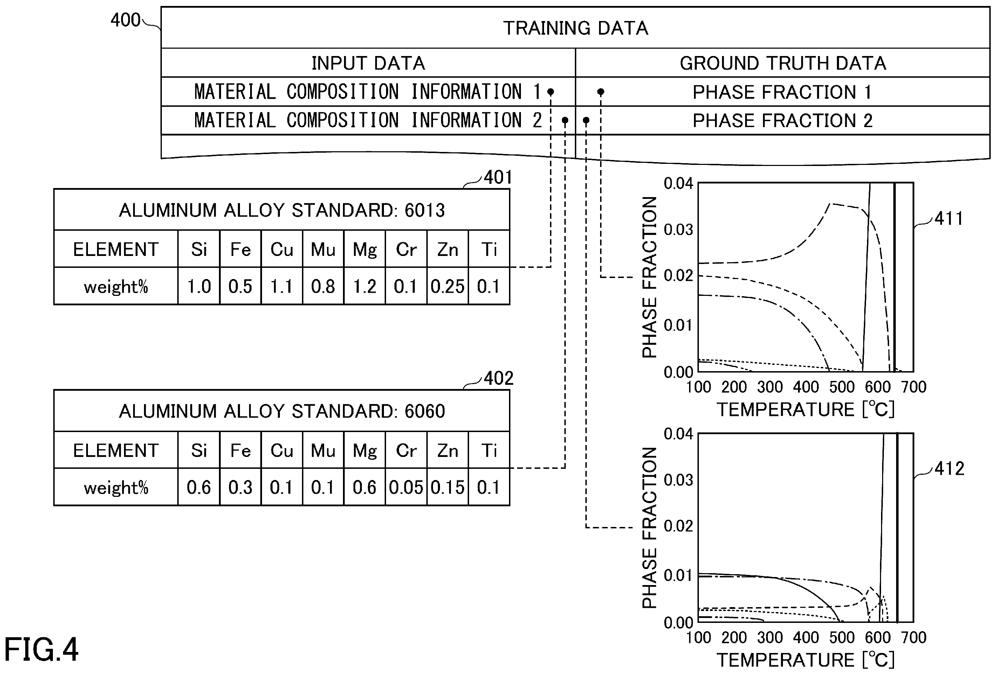

Next, a configuration of the training data and specific examples of the input data and the ground truth data included in the training data will be described. FIG. 4 is a diagram illustrating the configuration of the training data and the specific examples of the input data and the ground truth data.

As illustrated in FIG. 4, training data 400 includes "input data" and "ground truth data" as information items.

In "input data", "material composition information 1", "material composition information 2", ..., and the like, which are different material composition information, are stored. In FIG. 4, a reference symbol 401 indicates a specific example of "material composition information 1", and represents the weight% of each of the additive elements constituting "6013", which is the designation of the known aluminum alloy standard.

Similarly, a reference symbol 402 indicates a specific example of "material composition information 2", and represents the weight% of each of the additive elements constituting "6060", which is the designation of the known aluminum alloy standard.

In "ground truth data", "phase fraction 1", "phase fraction 2", ..., and the like, each of which is the phase fraction at each temperature within the predetermined temperature range (100°C to 700°C in the example of FIG. 4), associated with "material composition information 1", "material composition information 2", ..., and the like are stored. In FIG. 4, a reference symbol 411 indicates a specific example of "phase fraction 1", a reference symbol 412 indicates a specific example of "phase fraction 2", and in each case, the horizontal axis represents the temperature, and the vertical axis represents the phase fraction.

Here, the reference symbols 411 and 412 are results obtained by operating a simulator with high prediction accuracy and high cost for a long time, and in the present embodiment, these results are used as the ground truth data.

Here, the simulator described herein refers to software for calculating the phase fraction at each temperature within the predetermined temperature range by a thermodynamic equilibrium calculation, and include, for example, software such as CaTCalc (registered trademark) and MatCalc (registered trademark). Alternatively, the simulator may include software such as Termosuite (registered trademark), FactStage (registered trademark), and Pandat (registered trademark). Alternatively, the simulator may include software such as MALT2 (registered trademark), Thermo-Calc (registered trademark), and OpenCalphad (registered trademark).

### <Details of Each Unit of Training device>

Next, each unit (the training data generation unit 111 and the training unit 112) of the training device 110 will be described in detail.

### (1) Functional Configuration of Training data Generation Unit

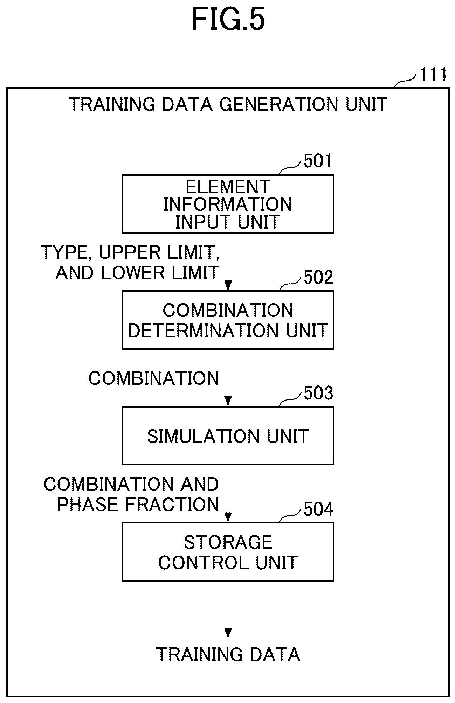

First, a functional configuration of the training data generation unit 111 will be described. FIG. 5 is a diagram illustrating an example of the functional configuration of the training data generation unit.

As illustrated in FIG. 5, the training data generation unit 111 includes an element information input unit 501, a combination determination unit 502, a simulation unit 503, and a storage control unit 504.

The element information input unit 501 receives, for example, an input of the type of an additive element to be added when a specific alloy is generated. Additionally, the element information input unit 501 receives an input of an upper limit value and a lower limit value of the addition amount (weight%) for each additive element that has been received as the input.

The combination determination unit 502 determines multiple combinations of the addition amounts of the respective additive elements by randomly selecting the addition amounts of the respective additive elements that have been received as the input under the constraint of the upper limit value and the lower limit value. Additionally, the combination determination unit 502 notifies the simulation unit 503 of the material composition information indicated by the determined multiple combinations.

The simulation unit 503 calculates the phase fraction at each temperature within the predetermined temperature range by operating the simulator described above for each of the multiple pieces of material composition information notified from the combination determination unit 502. Additionally, the simulation unit 503 notifies the storage control unit 504 of the multiple pieces of material composition information and corresponding phase fractions at the temperatures within the predetermined temperature range.

The storage control unit 504 generates the training data 400 in which the multiple pieces of material composition information notified from the simulation unit 503 are set as "input data" and the corresponding phase fractions at the temperatures within the predetermined temperature range are set as "ground truth data", and stores the training data 400 in the training data storage unit 113.

### (2) Functional Configuration of Training Unit

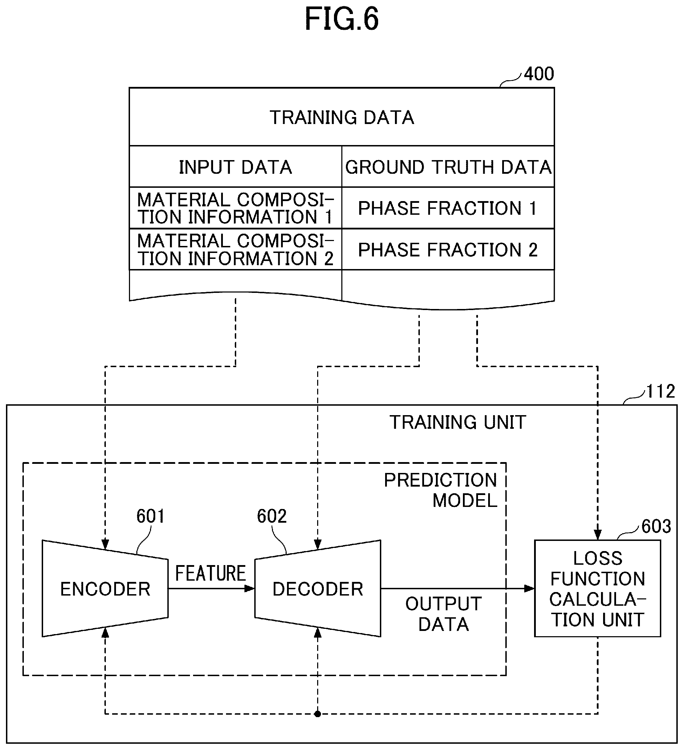

Next, a functional configuration of the training unit 112 will be described. FIG. 6 is a diagram illustrating an example of the functional configuration of the training unit.

As illustrated in FIG. 6, the training unit 112 includes the prediction model and a loss function calculation unit 603.

As described above, Seq2Seq with the attention mechanism; or Transformer is applied to the prediction model, and the prediction model includes an encoder 601 and a decoder 602.

The encoder 601 outputs a feature, by "material composition information 1", "material composition information 2", ..., and the like stored in "input data" of the training data 400 being input.

The decoder 602 sequentially outputs output data, by the feature output from the encoder 601 being input. Specifically, the decoder 602 reads "phase fraction 1", "phase fraction 2", ..., and the like stored in "ground truth data" of the training data 400. Then, the decoder 602 outputs the output data at the (i+1)-th temperature by using the phase fractions at the temperatures up to the i-th temperature (that is, the ground truth data at the temperatures up to the i-th temperature). With this, the decoder 602 can sequentially output the output data corresponding to the phase fraction at each temperature within the predetermined temperature range based on the feature output from the encoder 601 by "material composition information 1" being input and based on "phase fraction 1", which is "ground truth data", for example. Similarly, the decoder 602 can sequentially output the output data corresponding to the phase fraction at each temperature within the predetermined temperature range based on the feature output from the encoder 601 by "material composition information 2" being input and based on "phase fraction 2", which is "ground truth data", for example.

Here, it is assumed that a softmax function is used in an output layer of the decoder 602. With this, multiple values included in the output data of the decoder 602 (that is, values of the respective phases) are in a range of 0 to 1, and the sum of the multiple values (that is, the sum of the values of the respective phases) is "1".

As described, by using the softmax function in the output layer of the decoder 602, the output data corresponding to the phase fraction at each temperature within the predetermined temperature range can be sequentially output without performing additional calculation.

The loss function calculation unit 603 reads "phase fraction 1", "phase fraction 2", ..., and the like stored in "ground truth data" of the training data 400, and compares the read data with the output data at the (i+1)-th temperature output by the decoder 602.

Additionally, in the comparison, the loss function calculation unit 603 calculates multiple types of losses, and calculates an integration loss by performing weighted addition of the calculated multiple types of losses. Further, the loss function calculation unit 603 updates the model parameters of the encoder 601 and the decoder 602 based on the calculated integration loss. With this, the trained prediction model (including a trained encoder and a trained decoder) is generated.

### (3) Operation Example 1 of Training Unit

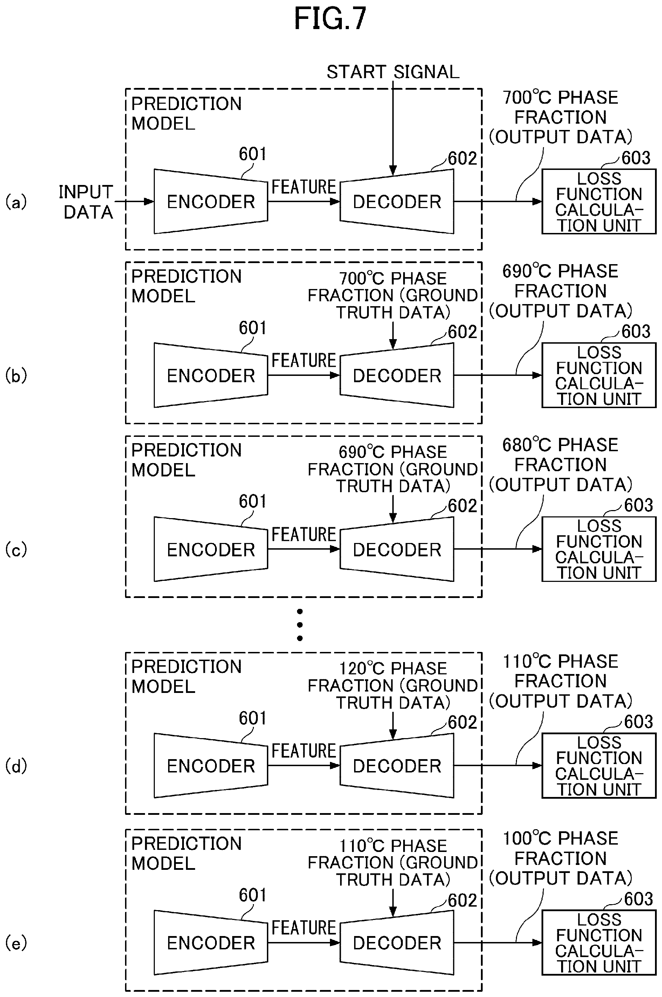

Next, an operation example (mainly, an operation example of the prediction model) of the training unit 112 will be described. FIG. 7 is a first diagram illustrating an operation example of the training unit.

Among these, FIG. 7 (a) illustrates a state in which the feature is output from the encoder 601 by "input data" of the training data 400 being read and input into the encoder 601. Additionally, FIG. 7 (a) illustrates a state in which the decoder 602 outputs output data by a start signal being input into the decoder 602. In the example of FIG. 7 (a), the output data output by the decoder 602 corresponds to the phase fraction at 700°C. Here, the start signal is a signal for starting the operation of the decoder 602, and in the present embodiment, an arbitrary value (for example, "000000" or the like) different from the output data output from the decoder 602 is input into the decoder 602 as the start signal. Additionally, the output data output from the decoder 602 (here, the output data corresponding to the phase fraction at 700°C) is notified to the loss function calculation unit 603.

FIG. 7 (b) illustrates a state in which the decoder 602 outputs output data by the feature output from the encoder 601 being input into the decoder 602 and the phase fraction at 700°C (the ground truth data at 700°C) being input into the decoder 602. In the example of FIG. 7 (b), the output data output by the decoder 602 corresponds to the phase fraction at 690°C. Here, the output data (here, the output data corresponding to the phase fraction at 690°C) output from the decoder 602 is notified to the loss function calculation unit 603.

FIG. 7 (c) illustrates a state in which the decoder 602 outputs output data by the feature output from the encoder 601 being input into the decoder 602 and the phase fractions down to 690°C (the ground truth data down to 690°C) being input into the decoder 602. However, in FIG. 7 (c), only the ground truth data at 690°C is illustrated because of space limitations (in practice, the ground truth data at 700°C and the ground truth data at 690°C are input into the decoder 602 with weights being applied thereto).

In the example of FIG. 7 (c), the output data output by the decoder 602 corresponds to the phase fraction at 680°C. Here, the output data (here, the output data corresponding to the phase fraction at 680°C) output from the decoder 602 is notified to the loss function calculation unit 603.

FIG. 7 (d) illustrates a state in which the decoder 602 outputs output data by the feature output from the encoder 601 being input into the decoder 602 and the phase fractions down to 120°C (the ground truth data down to 120°C) being input into the decoder 602. However, in FIG. 7 (d), only the ground truth data at 120°C is illustrated because of space limitations (in practice, each ground truth data from 700°C to 120°C is input into the decoder 602 with weights being applied thereto).

In the example of FIG. 7 (d), the output data output from the decoder 602 corresponds to the phase fraction at 110°C. Here, the output data (here, the output data corresponding to the phase fraction at 110°C) output from the decoder 602 is notified to the loss function calculation unit 603.

FIG. 7 (e) illustrates a state in which the decoder 602 outputs output data by the feature output from the encoder 601 being input into the decoder 602 and the phase fractions down to 110°C (the ground truth data down to 110°C.) being input into the decoder 602. However, in FIG. 7 (e), only the ground truth data at 110°C is illustrated because of space limitations (in practice, each ground truth data from 700°C to 110°C is input into the decoder 602 with weights being applied thereto).

In the example of FIG. 7 (e), the output data output by the decoder 602 corresponds to the phase fraction at 100°C. Here, the output data (here, the output data corresponding to the phase fraction at 100°C) output from the decoder 602 is notified to the loss function calculation unit 603.

### (4) Operation Example 2 of Training unit

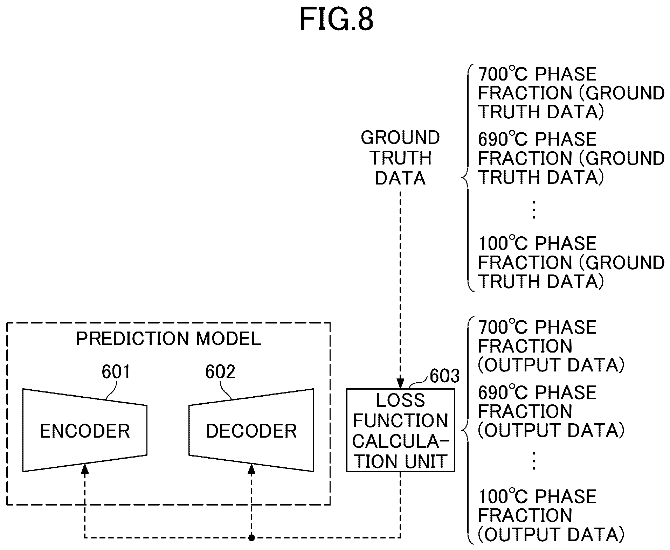

Next, an operation example (mainly, an operation example of the loss function calculation unit 603) of the training unit 112 will be described. FIG. 8 is a second diagram illustrating an operation example of the training unit. As illustrated in FIG. 8, the loss function calculation unit 603 reads "ground truth data" of the training data 400. The example of FIG. 8 illustrates a state in which the following phase fractions, which are the phase fractions at the respective temperatures within the predetermined temperature range included in "phase fraction 1", are read,
- the phase fraction at 700°C (ground truth data);
- the phase fraction at 690°C (ground truth data); ...; and
- the phase fraction at 100°C (ground truth data).

Additionally, the example of FIG. 8 illustrates a state in which as a result of sequentially outputting the output data from the decoder 602 based on the feature output from the encoder 601 by "material composition information 1" being input, the loss function calculation unit 603 holds:
- the phase fraction at 700°C (output data);
- the phase fraction at 690°C (output data); ...; and
- the phase fraction at 100°C (output data).

Additionally, the example of FIG. 8 illustrates a state in which the loss function calculation unit 603 compares the phase fractions (the ground truth data) at the respective temperatures of 100°C to 700°C with the phase fractions (output data) at the respective temperatures of 100°C to 700°C, and calculates the integration loss. Additionally, the example of FIG. 8 illustrates a state in which the loss function calculation unit 603 updates the model parameters of the encoder 601 and the decoder 602 based on the calculated integration loss. Here, the functional configuration of the loss function calculation unit 603 for calculating the integration loss will be described in detail below.

### (5) Details of Functional Configuration of Loss Function Calculation Unit

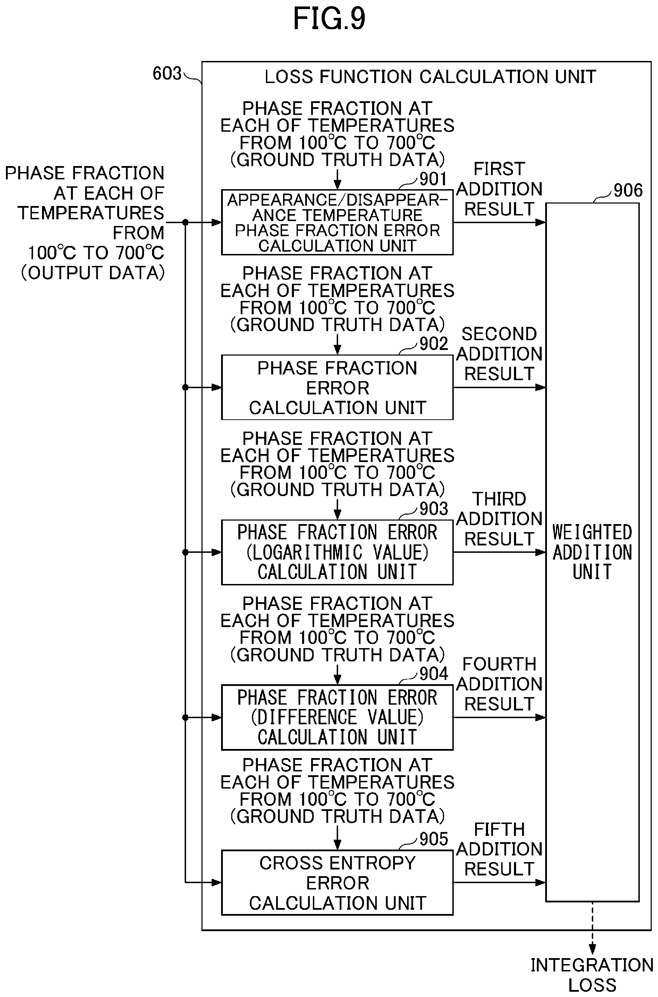

FIG. 9 is a diagram illustrating an example of a method of calculating the loss by the loss function calculation unit. As illustrated in FIG. 9, the loss function calculation unit 603 includes:
- an appearance/disappearance temperature phase fraction error calculation unit 901;
- a phase fraction error calculation unit 902;
- a phase fraction error (logarithmic value) calculation unit 903;
- a phase fraction error (difference value) calculation unit 904;
- a cross entropy error calculation unit 905; and
- a weighted addition unit 906,
as functions for calculating multiple types of losses.

With respect to a temperature at which the curve of each phase fraction becomes 0 in the ground truth data of the training data, the appearance/disappearance temperature phase fraction error calculation unit 901 compares the phase fraction (the ground truth data) at the temperature of 100°C to 700°C with the phase fraction (the output data) at the temperature of 100°C to 700°C. With this, the appearance/disappearance temperature phase fraction error calculation unit 901 adds the error between a phase fraction determined based on the output data and a phase fraction determined based on the training data, at the temperature, determined based on the training data, at which a phase appears or disappears, for all phases, and outputs a result of the addition (a first addition result).

The phase fraction error calculation unit 902 compares the phase fractions (the ground truth data) at the respective temperatures of 100°C to 700°C with the phase fractions (the output data) at the respective temperatures of 100°C to 700°C. With this, the phase fraction error calculation unit 902 adds the errors the phase fractions at the respective temperatures for the predetermined temperature range and outputs a result of the addition (a second addition result).

The phase fraction error (logarithmic value) calculation unit 903 compares the phase fractions (the ground truth data) at the respective temperatures of 100°C to 700°C with the phase fractions (the output data) at the respective temperatures of 100°C to 700°C. With this, the phase fraction error (logarithmic value) calculation unit 903 adds the errors of the logarithmic values of the phase fractions at the respective temperatures within the predetermined temperature range for the predetermined temperature range, and outputs a result of the addition (a third addition result).

The phase fraction error (difference value) calculation unit 904 compares the phase fractions (the ground truth data) at the respective temperatures of 100°C to 700°C with the phase fractions (the output data) at the respective temperatures of 100°C to 700°C. With this, the phase fraction error (difference value) calculation unit 904 adds the error of the difference value between the phase fractions at the adjacent temperatures, for the predetermined temperature range, and outputs a result of the addition (a fourth addition result).

The cross entropy error calculation unit 905 compares the phase fractions (the ground truth data) at the respective temperatures of 100°C to 700°C with the phase fractions (the output data) at the respective temperatures of 100°C to 700°C. With this, the cross entropy error calculation unit 905 adds the error of the ratios of the phase fractions at each temperature within the predetermined temperature range, for the predetermined temperature range, and outputs a result of the addition (a fifth addition result).

The weighted addition unit 906 calculates the integration loss by weighting and adding the results of the addition (the first addition result to the fifth addition result) output from the appearance/disappearance temperature phase fraction error calculation unit 901 to the cross entropy error calculation unit 905.

As described, by using a configuration in which multiple types of losses are calculated, and are weighted and added, according to the loss function calculation unit 603, the loss between the output data and the ground truth data is processed in multiple aspects. As a result, the training unit 112 can appropriately update the model parameters when training the prediction model.

### <Flow of Training Process>

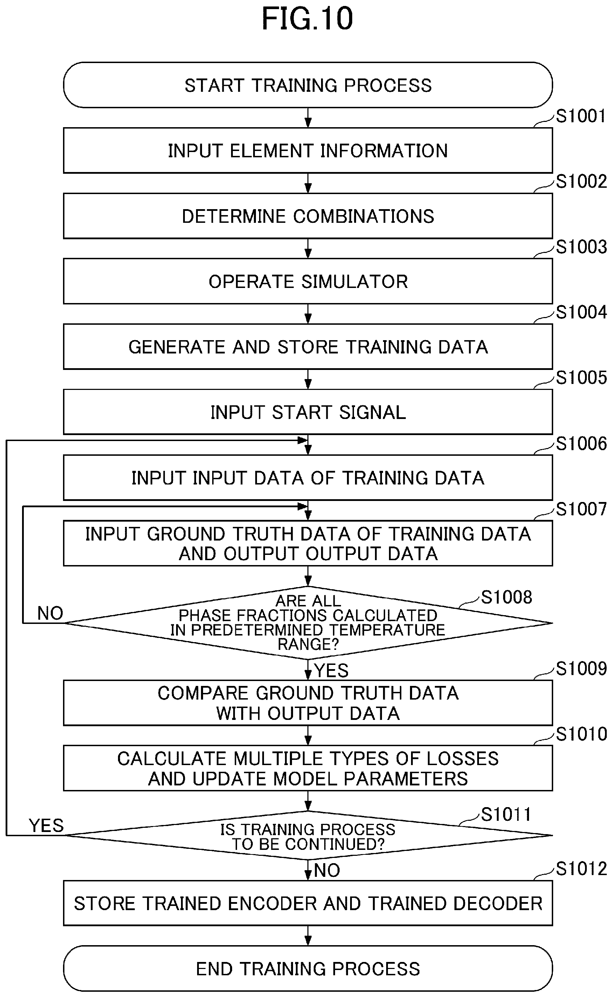

Next, a flow of the training process by the training device 110 will be described. FIG. 10 is a flowchart illustrating the flow of the training process.

In step S1001, the training data generation unit 111 receives, as the element information, an input of the types of the additive elements to be added when generating the specific alloy, and the upper limit value and the lower limit value of the addition amount (weight%) of each of the additive elements.

In step S1002, the training data generation unit 111 determines multiple combinations of the addition amounts of the respective additive elements by randomly selecting the addition amounts of the additive elements under the constraint of the upper limit value and the lower limit value.

In step S1003, the training data generation unit 111 causes the simulator to operate for each of the material composition information indicated by the determined multiple combinations, and calculates the respective phase fractions at the temperatures within the predetermined temperature range.

In step S1004, the training data generation unit 111 generates the training data and stores the training data in the training data storage unit 113.

In step S1005, the training unit 112 inputs the start signal to the decoder 602.

In step S1006, the training unit 112 reads "input data" of the training data and inputs "input data" into the prediction model.

In step S1007, the training unit 112 sequentially inputs the phase fractions of "ground truth data" of the training data at the temperatures up to the i-th temperature (that is, the ground truth data at the temperatures up to the i-th temperature) to sequentially output the (i+1)-th output data.

In step S1008, the training unit 112 determines whether all output data corresponding to the phase fractions at the respective temperatures within the predetermined temperature range have been output. If it is determined in step S1008 that there is a temperature at which the output data corresponding to the phase fraction is not output (NO in step S1008), the process returns to step S1007.

When it is determined in step S1008 that all the output data corresponding to the phase fractions at the respective temperatures within the predetermined temperature range have been output (YES in step S1008), the process proceeds to step S1009.

In step S1009, the training unit 112 reads "ground truth data" of the training data and compares "ground truth data" with the output data.

In step S1010, the training unit 112 calculates multiple types of losses, and weights and adds the calculated multiple types of losses to calculate the integration loss. Additionally, the training unit 112 updates the model parameters of the encoder 601 and the decoder 602 based on the calculated integration loss.

In step S1011, the training unit 112 determines whether to continue the training process. If it is determined in step S1011 that the training process is to be continued (YES in step S1011), the process returns to step S1006, and substantially the same process is performed by reading the next "input data" of the training data.

When it is determined in step S1011 that the training process is to be ended (NO in step S1011), the process proceeds to step S1012.

In step S1012, the training unit 112 stores the trained encoder and the trained decoder in the prediction device 120 and the composition optimization device 130 as the trained prediction model.

### <Details of Each Unit of Prediction Device>

Next, each unit (here, the prediction unit 122) of the prediction device 120 will be described in detail.

### (1) Functional Configuration of Prediction Unit

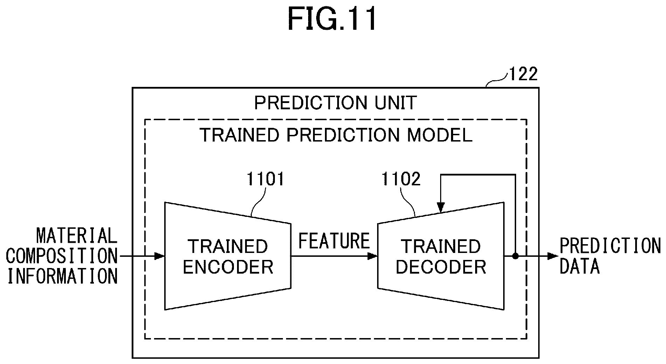

First, a functional configuration of the prediction unit 122 will be described. FIG. 11 is a diagram illustrating an example of the functional configuration of the prediction unit.

As illustrated in FIG. 11, the prediction unit 122 includes a trained encoder 1101 and a trained decoder 1102 that have been trained by the training unit 112. The trained encoder 1101 and the trained decoder 1102 form the trained prediction model.

The trained encoder 1101 calculates a feature by the material composition information (the material composition information on the prediction target material) notified from the material composition input unit 121 being input, and outputs the calculated feature to the trained decoder 1102.

The trained decoder 1102 sequentially outputs prediction data by the feature output from the trained encoder 1101 being input. Specifically, the trained decoder 1102 outputs the prediction data at the (i+1)-th temperature by using the prediction data at the temperatures up to the i-th temperature. This allows the trained decoder 1102 to predict the phase fraction at each temperature within the predetermined temperature range based on the feature output from the trained encoder 1101 by the material composition information on the prediction target material being input.

### (2) Operation Example of Prediction Unit

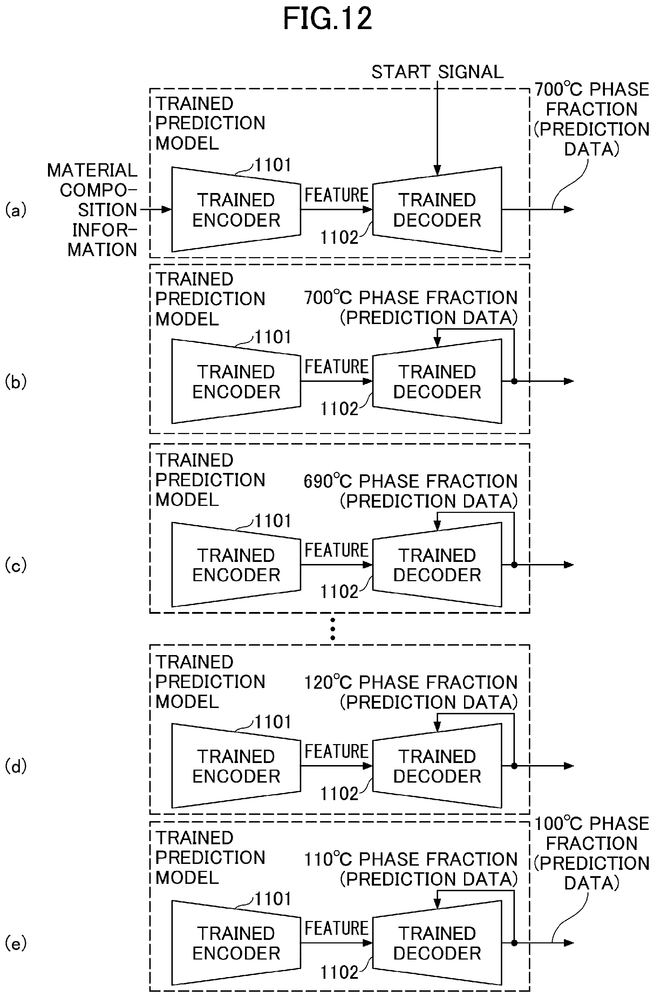

Next, an operation example of the prediction unit 122 will be described. FIG. 12 is a diagram illustrating the operation example of the prediction unit. FIG. 12 (a) illustrates a state in which the feature is output from the trained encoder 1101 by the material composition information on the prediction target material being input into the trained encoder 1101. Additionally, FIG. 12 (a) illustrates a state in which the prediction data (the phase fraction at 700°C) is output by the feature output from the trained encoder 1101 being input into the trained decoder 1102 and the start signal being input into the trained decoder 1102.

FIG. 12 (b) illustrates a state in which the feature output from the trained encoder 1101 is input into the trained decoder 1102 and the prediction data (the phase fraction at 700°C) is input into the trained decoder 1102. WIth this, the trained decoder 1102 outputs the prediction data (the phase fraction at 690°C).

FIG. 12 (c) illustrates a state in which the feature output from the trained encoder 1101 is input into the trained decoder 1102 and the prediction data (the phase fractions down to 690°C) is input into the trained decoder 1102. Here, in FIG. 12 (c), only the phase fraction at 690°C is illustrated because of space limitations (in practice, the phase fraction at 700°C and the phase fraction at 690°C are input into the trained decoder 1102 with weights being applied thereto). With this, the trained decoder 1102 outputs the prediction data (the phase fraction at 680°C).

FIG. 12 (d) illustrates a state in which the feature output from the trained encoder 1101 is input into the trained decoder 1102 and the prediction data (the phase fractions down to 120°C) is input into the trained decoder 1102. Here, in FIG. 12 (d), only the phase fraction at 120°C is illustrated because of space limitations (in practice, the phase fractions from 700°C to 120°C are input into the trained decoder 1102 with weights being applied thereto). With this, the trained decoder 1102 outputs the prediction data (the phase fraction at 110°C).

FIG. 12 (e) illustrates a state in which the feature output from the trained encoder 1101 is input into the trained decoder 1102 and the prediction data (the phase fractions down to 110°C) is input into the trained decoder 1102. Here, in FIG. 12 (e), only the phase fraction at 110°C is illustrated because of space limitations (in practice, the phase fractions from 700°C to 110°C are input into the trained decoder 1102 with weights being applied thereto). With this, the trained decoder 1102 outputs the prediction data (the phase fraction at 100°C). In the example of FIG. 12 (e), the prediction data (the phase fraction at 100°C) output from the trained decoder 1102 by the feature and the prediction data (the phase fractions down to 110°C) being input is also illustrated.

### <Flow of Prediction Process>

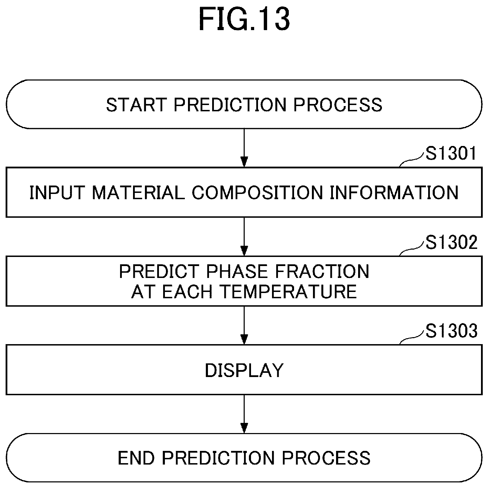

Next, a flow of a prediction process by the prediction device 120 will be described. FIG. 13 is a flowchart illustrating the flow of the prediction process.

In step S1301, the material composition input unit 121 receives an input of the material composition information on the prediction target material.

In step S1302, the prediction unit 122 receives the start signal and inputs the input material composition information into the trained prediction model, to predict the phase fraction at each temperature within the predetermined temperature range.

In step S1303, the display unit 123 displays the predicted phase fraction at each temperature.

### <Prediction Accuracy>

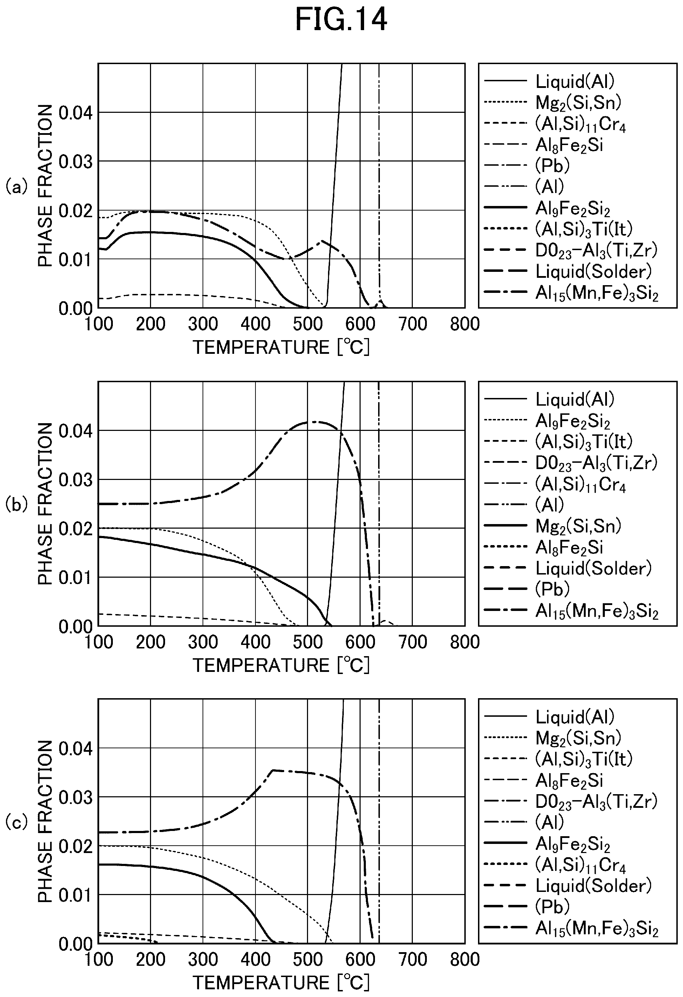

Next, the prediction accuracy of the phase fraction at each temperature within the predetermined temperature range predicted by the prediction unit 122 will be described. FIG. 14 depicts graphs for explaining the prediction accuracy. FIGS. 14 (a) to (c) each indicate the prediction data in the case where the weight% of each additive element constituting "6013", which is the designation of the known aluminum alloy standard, is input as the material composition information on the prediction target material. Here, in the present embodiment, the ground truth data indicated by the reference symbol 411 in FIG. 4 is used to evaluate the prediction accuracy. The loss from the ground truth data is calculated by using a mean squared logarithmic error (MSLE) loss.

FIG. 14 (a) illustrates prediction data in a case where a fully connected multilayer neural network is applied to the prediction model as a comparative example. In FIG. 14 (a), the MSLE loss from the ground truth data is 3.79 × 10⁻⁴.

FIG. 14 (b) illustrates prediction data when Seq2Seq with the attention mechanism is applied to the prediction model. In FIG. 14 (b), the MSLE loss from the ground truth data is 5.1 × 10⁻⁵.

FIG. 14 (c) illustrates prediction data in a case where Transformer is applied to the prediction model. In FIG. 14 (c), the MSLE loss from the ground truth data is 2.45 × 10⁻⁵.

As described, when the fully connected multilayer neural network is applied as the prediction model for realizing the calculation of the phase fraction at low cost, the prediction accuracy is low in some temperature regions (for example, 400°C to 600°C).

When Seq2Seq with the attention mechanism is applied as the prediction model for realizing the calculation of the phase fraction at low cost, the loss can be significantly improved. Further, when Transfomer is applied, the phase fraction of substantially the same level as the ground truth data can be reproduced.

That is, when predicting the phase fractions over the predetermined temperature range based on the material composition information, the prediction accuracy can be improved by:
- processing the phase fraction at each temperature as the time series data by using a recursive network;
- processing the loss in multiple aspects by using multiple types of loss functions.

### <Details of Process in Composition Optimization Device>

Next, a process in the composition optimization device 130 will be described in detail.

### (1) First Details of Forward Propagation Process in Composition Optimization Device

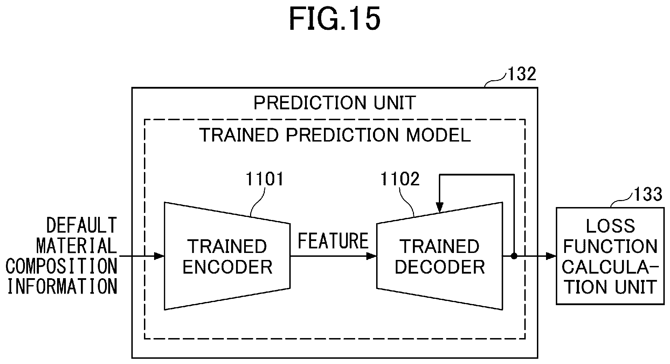

First, details of a forward propagation process in the composition optimization device 130 (mainly details of a process in the prediction unit 132) will be described. FIG. 15 is a first diagram illustrating an operation example of the forward propagation process in the composition optimization device.

As illustrated in FIG. 15, the trained encoder 1101 and the trained decoder 1102 that have been trained by the training unit 112 are included in the prediction unit 132, as in the prediction unit 122. The trained encoder 1101 and the trained decoder 1102 form the trained prediction model.

In the forward propagation process in the composition optimization device 130, the trained encoder 1101 calculates the feature by the default material composition information notified from the material composition input unit 131 being input, and outputs the calculated feature to the trained decoder 1102.

Additionally, in the forward propagation process in the composition optimization device 130, the trained decoder 1102 sequentially outputs the prediction data by the feature output from the trained encoder 1101 being input. Specifically, the trained decoder 1102 outputs the prediction data at the (i+1)-th temperature by using the prediction data at the temperatures up to the i-th temperature. As described, the trained decoder 1102 can predict the phase fraction at each temperature within the predetermined temperature range based on the feature output from the trained encoder 1101 by the default material composition information being input. Here, in the forward propagation process in the composition optimization device 130, the prediction data output from the trained decoder 1102 is input into the loss function calculation unit 133.

### (2) Second Details of Forward Propagation Process in Composition Optimization Device

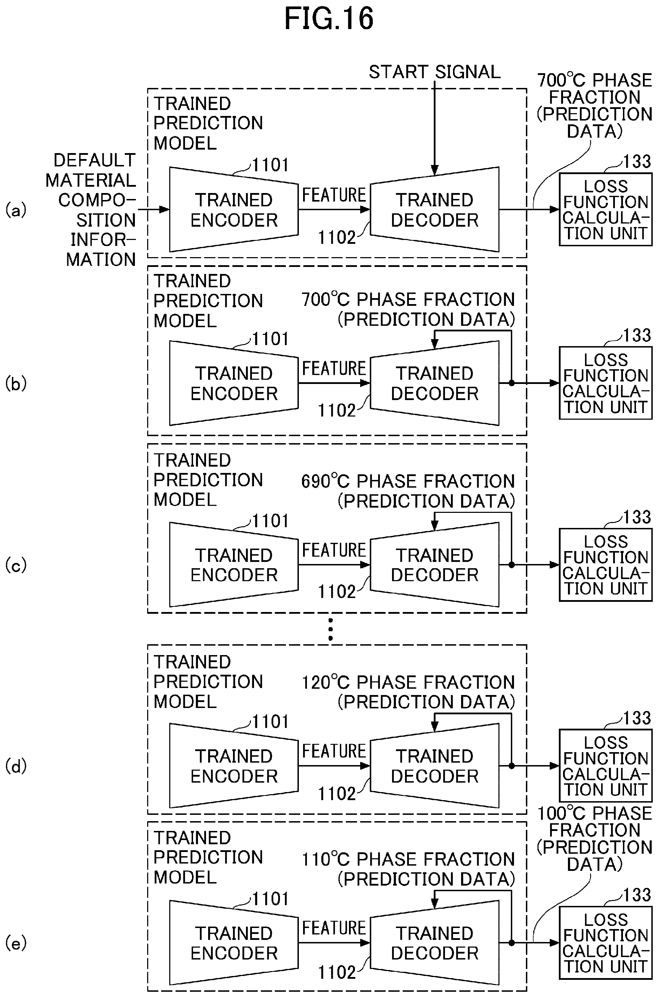

Next, further details of the forward propagation process in the composition optimization device 130 (mainly, further details of the process in the prediction unit 132) will be described. FIG. 16 is a second diagram illustrating the operation example of the forward propagation process in the composition optimization device. FIG. 16 (a) illustrates a state in which the feature is output from the trained encoder 1101 by the default material composition information being input into the trained encoder 1101. Additionally, FIG. 16 (a) illustrates a state in which the prediction data (the phase fraction at 700°C) is output by the feature output from the trained encoder 1101 being input into the trained decoder 1102 and the start signal being input into the trained decoder 1102. Further, FIG. 16 (a) illustrates a state in which the output prediction data (the phase fraction at 700°C) is input into the loss function calculation unit 133.

FIG. 16 (b) illustrates a state in which the feature output from the trained encoder 1101 is input into the trained decoder 1102 and the prediction data (the phase fraction at 700°C) is input into the trained decoder 1102. With this, the trained decoder 1102 outputs the prediction data (the phase fraction at 690°C). Here, the prediction data (the phase fraction at 690°C) output from the trained decoder 1102 is input into the loss function calculation unit 133.

FIG. 16 (c) illustrates a state in which the feature output from the trained encoder 1101 is input into the trained decoder 1102 and the prediction data (the phase fractions down to 690°C) is input into the trained decoder 1102. Here, in FIG. 16 (c), only the phase fraction at 690°C is illustrated because of space limitations (in practice, the phase fraction at 700°C and the phase fraction at 690°C are input into the trained decoder 1102 with weights being applied thereto). With this, the trained decoder 1102 outputs the prediction data (the phase fraction at 680°C). Here, the prediction data (the phase fraction at 680°C) output from the trained decoder 1102 is input into the loss function calculation unit 133.

FIG. 16 (d) illustrates a state in which the feature output from the trained encoder 1101 is input into the trained decoder 1102 and the prediction data (the phase fractions down to 120°C) is input into the trained decoder 1102. Here, in FIG. 16 (d), only the phase fraction at 120°C is illustrated because of space limitations (in practice, the phase fractions from 700°C to 120°C are input into the trained decoder 1102 with weights being applied thereto). With this, the trained decoder 1102 outputs the prediction data (the phase fraction at 110°C). Here, the prediction data (the phase fraction at 120°C) output from the trained decoder 1102 is input into the loss function calculation unit 133.

FIG. 16 (e) illustrates a state in which the feature output from the trained encoder 1101 is input into the trained decoder 1102 and the prediction data (the phase fractions down to 110°C) is input into the trained decoder 1102. Here, in FIG. 16 (e), only the phase fraction at 110°C is illustrated because of space limitations (in practice, the phase fractions from 700°C to 110°C are input into the trained decoder 1102 with weights being applied thereto). With this, the trained decoder 1102 outputs the prediction data (the phase fraction at 100°C). Here, the example of FIG. 16 (e) also illustrates a state in which the prediction data (the phase fraction at 100°C) is output from the trained decoder 1102 by the feature and the prediction data (the phase fractions down to 110°C) being input, and is input into the loss function calculation unit 133.

### (3) Third Details of Forward Propagation Processing in Composition Optimization Device

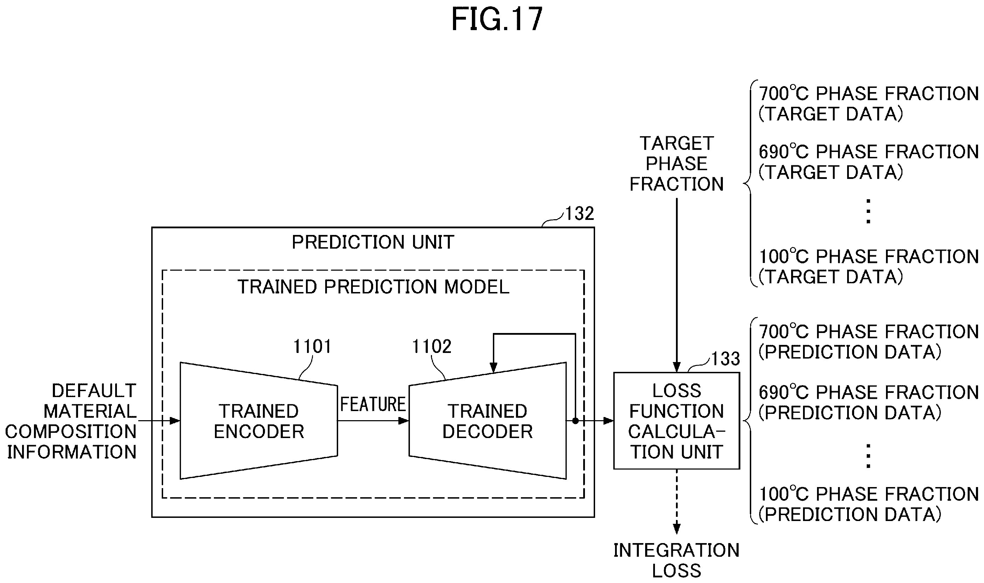

Next, details of the forward propagation process in the composition optimization device 130 (mainly details of the process in the loss function calculation unit 133) will be described. FIG. 17 is a third diagram illustrating an operation example of the forward propagation process in the composition optimization device.

As illustrated in FIG. 17, in the forward propagation process in the composition optimization device 130, the target phase fraction is set in advance in the loss function calculation unit 133. The example of FIG. 17 illustrates a state in which the following phase fractions are set as the target phase fraction:
- the phase fraction at 700°C (the target data);
- the phase fraction at 690°C (the target data); ...; and
- the phase fraction at 100°C (the target data).

Additionally, as described above, in the forward propagation process in the composition optimization device 130, the following phase fractions are input into the loss function calculation unit 133 as the phase fraction at each temperature within the predetermined temperature range output from the trained decoder 1102:
- the phase fraction at 700°C (the prediction data);
- the phase fraction at 690°C (the prediction data); ...; and
- the phase fraction at 100°C (the prediction data).

Additionally, in the forward propagation process in the composition optimization device 130, the loss function calculation unit 133 calculates the integration loss based on the phase fractions (the target data and the prediction data) at each temperature within the predetermined temperature range. The example of FIG. 17 illustrates a state in which the integration loss is calculated by comparing the target phase fractions (the target data) at the respective temperatures of 100°C to 700°C and the predicted phase fractions (the prediction data) at the respective temperatures of 100°C to 700°C.

### (4) Details of Backpropagation Process in Composition Optimization Device

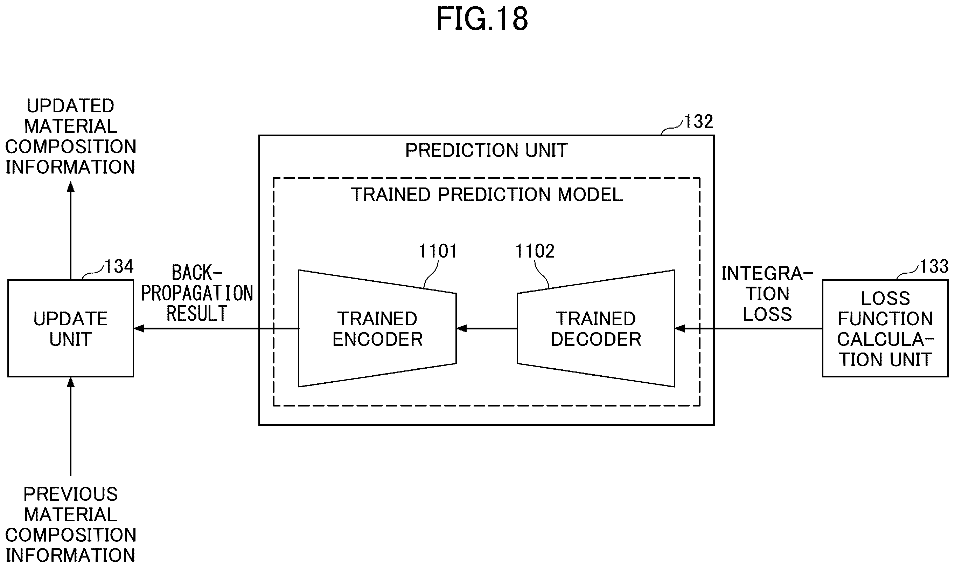

Next, the details of the backpropagation process in the composition optimization device 130 (mainly, the details of the processes in the loss function calculation unit 133, the prediction unit 132, and the update unit 134) will be described. FIG. 18 is a diagram illustrating an operation example of the backpropagation process in the composition optimization device.

As illustrated in FIG. 18, in the backpropagation process in the composition optimization device 130, the loss function calculation unit 133 performs backpropagation of the integration loss corresponding to the default composition material information in a state in which the model parameters of the trained prediction model of the prediction unit 132 are fixed. With this, the backpropagation result is output from the trained prediction model of the prediction unit 132 and input into the update unit 134.

Additionally, as illustrated in FIG. 18, in the backpropagation process in the composition optimization device 130, the update unit 134 updates the material composition information based on the backpropagation result output from the prediction unit 132 and the previous material composition information (here, the default material composition information). With this, the update unit 134 generates the updated material composition information.

### (5) Fourth Details of Forward Propagation Process in Composition Optimization Device

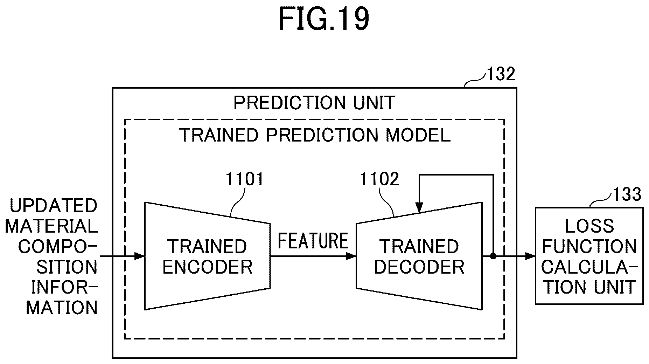

Next, details of the forward propagation process in the composition optimization device 130 (mainly details of the process in the prediction unit 132) will be described. FIG. 19 is a fourth diagram illustrating an operation example of the forward propagation process in the composition optimization device. The difference from FIG. 15 is that, in FIG. 19, the updated material composition information generated by the update unit 134 is input into the trained encoder 1101. Here, the forward propagation process and the backward propagation process after the updated material composition information is input into the trained encoder 1101 are the same as the forward propagation process and the backward propagation process described in FIG. 16 to FIG. 18, and thus the description thereof will be omitted here.

### <Flow of Composition Optimization Process>

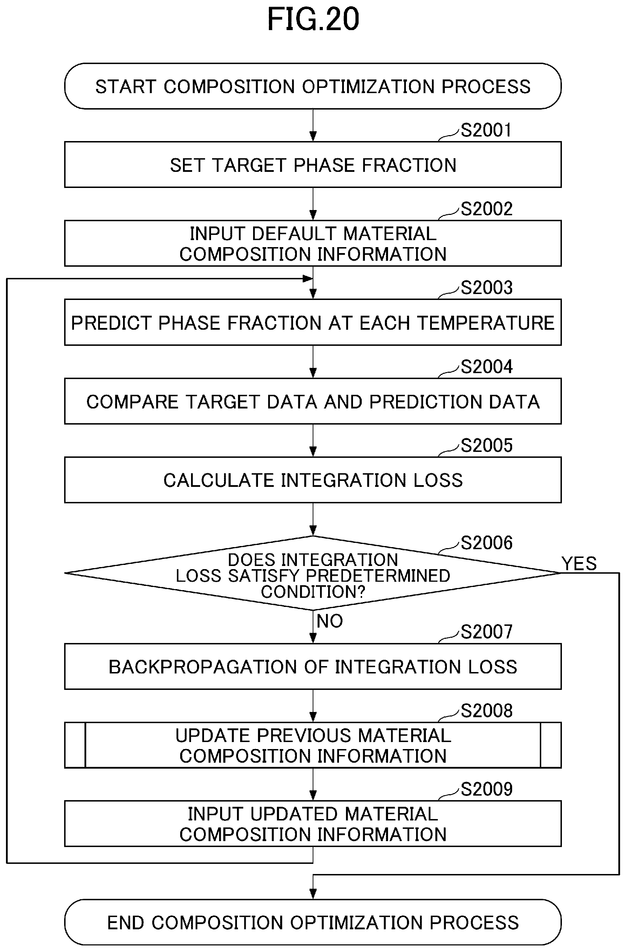

Next, a flow of the composition optimization process by the composition optimization device 130 will be described. FIG. 20 is a flowchart illustrating the flow of the composition optimization process.

In step S2001, the loss function calculation unit 133 receives a setting of the target phase fraction.

In step S2002, the material composition input unit 131 receives an input of the default material composition information.

In step S2003, the prediction unit 132 receives the start signal and inputs the input material composition information into the trained prediction model to output the predicted phase fraction (the prediction data) at each temperature within the predetermined temperature range.

In step S2004, the loss function calculation unit 133 compares the predicted phase fractions (the prediction data) output from the trained prediction model with the target phase fractions (the target data).

In step S2005, the loss function calculation unit 133 calculates the integration loss as a result of the comparison.

In step S2006, the loss function calculation unit 133 determines whether the integration loss satisfies a predetermined condition. When it is determined in step S2006 that the predetermined condition is not satisfied (NO in step S2006), the process proceeds to step S2007.

In step S2007, the prediction unit 132 performs backpropagation of the calculated integration loss in a state where the model parameters of the trained prediction model are fixed, and outputs a result of the backpropagation.

In step S2008, the update unit 134 updates the previous material composition information based on the output result of the backpropagation. Here, the process of updating the previous material composition information will be described in detail later.

In step S2009, the material composition input unit 131 receives an input of the updated material composition information, and then the process returns to step S2003.

When it is determined in step S2006 that the predetermined condition is satisfied (YES in step S2006), the material composition information when it is determined that the predetermined condition is satisfied is set as a search result, and the composition optimization process is ended.

### <Details of Material Composition Update Process>

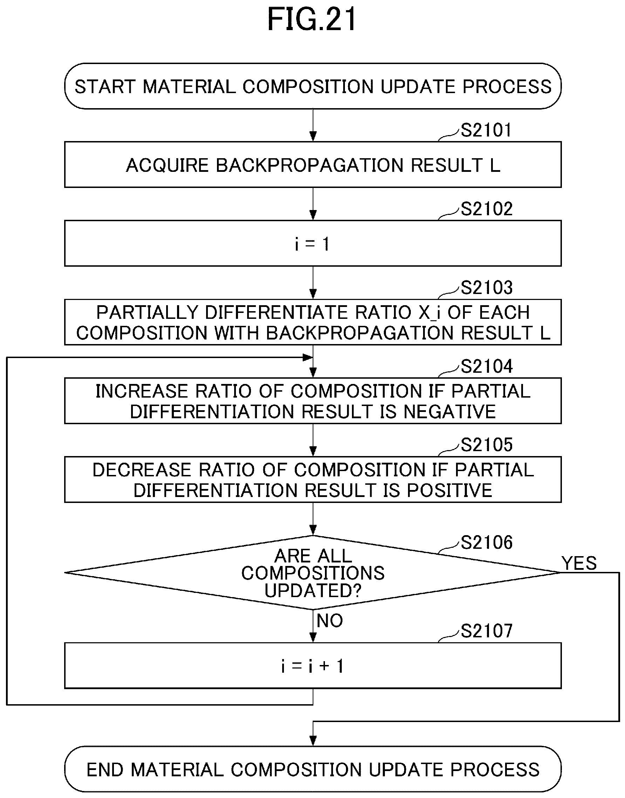

Next, the process of updating the previous material composition information (step S2008) will be described in detail. FIG. 21 is a flowchart illustrating a flow of the material composition update process.

In step S2101, the update unit 134 acquires the backpropagation result L output from the trained prediction model of the prediction unit 132.

In step S2102, the update unit 134 inputs "1" to a counter I.

In step S2103, the update unit 134 partially differentiates the ratio X_i of each composition of the previous material composition information with respect to the backpropagation result L. Here, the result of partial differentiation of the ratio X_i of each composition with respect to the backpropagation result L is a value indicating whether the error increases or decreases when the ratio X_i of the corresponding composition increases. The update unit 134 increases or decreases the ratio X_i of each composition so that the integration loss is reduced.

In step S2104, the update unit 134 increases the ratio X_i of the corresponding composition if the result of the partial differentiation in step S2103 is a negative value. Here, the result of the partial differentiation being the negative value indicates that the error decreases as the ratio X_i of the corresponding composition increases.

In step S2105, the update unit 134 decreases the ratio X_i of the corresponding composition if the result of the partial differentiation in step S2103 is a positive value. Here, the result of the partial differentiation being the positive value indicates that the error increases as the ratio X_i of the corresponding composition increases.

In step S2106, the update unit 134 determines whether all the compositions included in the previous material composition information have been updated.

When it is determined in step S2106 that there is a composition that is not updated (NO in step S2106), the process proceeds to step S2107.

In step S2107, the update unit 134 increments the counter I, and then the process returns to step S2104.

When it is determined in step S2106 that all the compositions have been updated (YES in step S2106), the material composition update process is ended.

### <Processing Result of Composition Optimization process>

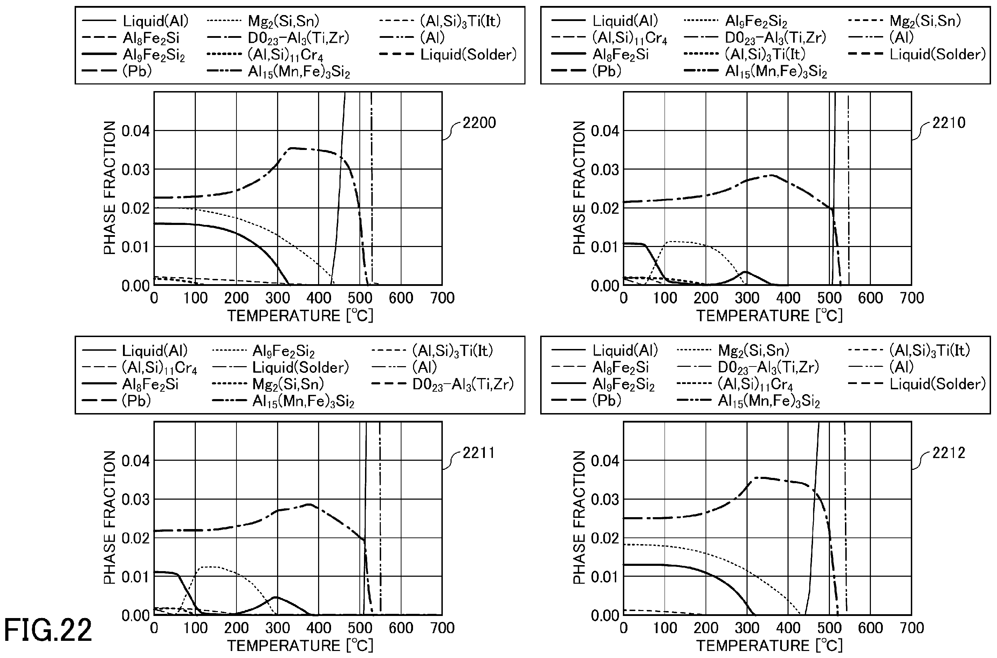

Next, a processing result of the composition optimization process by the composition optimization device 130 will be described. FIG. 22 depicts first graphs indicating examples of the processing result of the composition optimization process.

In FIG. 22, a reference symbol 2200 is an example of the phase fraction corresponding to the default material composition information that is input into the prediction unit 132.

Additionally, in FIG. 22, a reference symbol 2210 is an example of the target phase fraction set in the loss function calculation unit 133.

Additionally, in FIG. 22, a reference symbol 2211 indicates the predicted phase fraction in a case where the composition optimization device 130 according to the present embodiment performs the composition optimization process on the target phase fraction so that the integration loss satisfies the predetermined condition. Specifically, the reference symbol 2211 indicates the predicted phase fraction output by inputting, into the prediction unit 132, the updated material composition information obtained by updating the material composition information input into the prediction unit 132, 515 times.

In FIG. 22, a reference symbol 2212 indicates the predicted phase fraction in a case where the composition optimization process is performed by calculating only the RMSLE when the loss function calculation unit 133 calculates the integration loss, as a comparative example. Specifically, the reference symbol 2212 indicates the predicted phase fraction output by inputting, into the prediction unit 132, the updated material composition information obtained by updating the material composition information input into the prediction unit 132, 800 times. Here, RMSLE is an abbreviation of a root mean squared logarithmic error, and indicates a square root of a mean square logarithmic error.

As is clear from the comparison between the reference symbol 2210 and the reference symbol 2211, according to the composition optimization device 130 of the present embodiment, a material composition having predicted phase fractions substantially the same as the target phase fractions has been found at the stage where the material composition information has been updated 515 times.

With respect to the above, as is clear from the comparison between the reference symbol 2210 and the reference symbol 2212, when an appropriate loss is not calculated by the loss function calculation unit 133, even when the material composition information has been updated 800 times, a material composition having predicted phase fractions substantially the same as the target phase fractions has not been searched for yet.

As described, if an appropriate loss is not calculated when the integration loss is calculated, it takes time to search for the material composition information satisfying the predetermined condition with respect to the target phase fraction. On the contrary, if an appropriate loss is calculated when the integration loss is calculated, the time required for searching for the material composition information satisfying the predetermined condition with respect to the target phase fraction can be shortened.

### <Summary>

As is clear from the above description, the material development support system 100 according to the first embodiment is configured to:
- perform training using the training data in which the material composition information on the training target material and the phase fraction at each temperature of the training target material within the predetermined temperature range are associated with each other, to generate the trained model;
- input the default material composition into the generated trained model to predict the phase fraction at each temperature of the material having the predetermined material composition within the predetermined temperature range;
- perform backpropagation of the integration loss calculated based on the target phase fraction (the target data) and the predicted phase fraction (the prediction data) to update the predetermined material composition input into the trained model; and
- repeat the process of outputting the predicted phase fraction (the prediction data) based on the updated material composition and a process of updating the default material composition by performing backpropagation of the calculated integration loss until the integration loss satisfies the predetermined condition.

As described, in the material development support system 100 according to the first embodiment, when a material composition corresponding to the target phase fraction is searched for, instead of solving a huge number of forward problems with exhaustively changing the material composition, the material composition is changed by performing backpropagation of the integration loss.

With this, according to the material development support system 100 of the first embodiment, the material composition information corresponding to the target phase fraction can be efficiently searched for.

### [Second Embodiment]

In the first embodiment described above, the description is provided as freely updating the previous material composition information by using the backpropagation result output in the backpropagation process. With respect to the above, the material composition information is information indicating the ratio of each composition, and therefore, the material composition information needs to be always a positive value and the total value needs to be 100% (1.0). Therefore, in a second embodiment, the updated material composition information is corrected so that the updated material composition information is always a positive value and the total value is 100% (1.0). In the following, the second embodiment will be described, focusing on the differences from the first embodiment.

### <Functional Configuration of Composition Optimization Device>

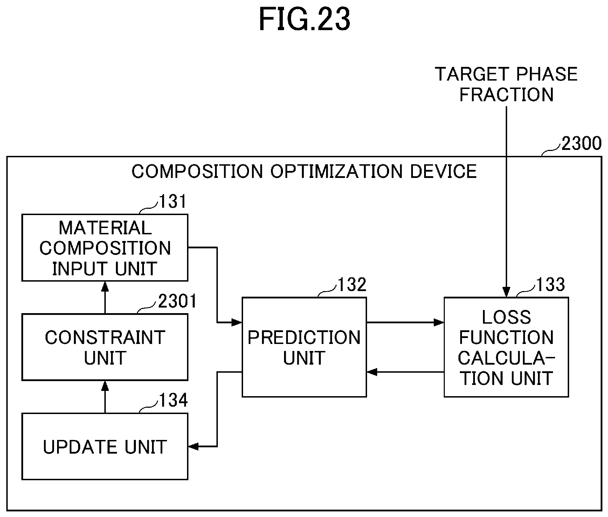

First, a functional configuration of a composition optimization device according to the second embodiment will be described. FIG. 23 is a first diagram illustrating another example of the functional configuration of the composition optimization device. The difference from the functional configuration of the composition optimization device 130 described with reference to FIG. 1 is that a composition optimization device 2300 according to the second embodiment includes a constraint unit 2301.

The constraint unit 2301 corrects the updated material composition information so that the total value of the ratios of the respective compositions becomes 100%. For example, when the material is an aluminum alloy, the ratio of the aluminum element is calculated by subtracting the total of the ratios of the elements other than the aluminum element from 100%. This allows the constraint unit 2301 to set the total value of the ratios of the respective compositions to 100% with respect to the updated material composition information.

Additionally, when the ratio X_i of the composition becomes a negative value as a result of the update unit 134 decreasing the ratio X_i of the composition, the constraint unit 2301 corrects the ratio X_i of the composition to zero, thereby avoiding the ratio X_i of the composition from becoming a negative value. That is, the constraint unit 2301 corrects the updated material composition information so that the ratio of each composition is 0% or greater.

Here, the updated material composition information may be used without the difference between the total value of the ratios of the respective compositions and 100% being adjusted, or may be adjusted by uniformly decreasing the ratios of the compositions other than the ratio X_i of the composition, for example. Alternatively, the updated material composition information may be adjusted by decreasing the largest ratio of the composition so that the total value of the ratios of the respective compositions becomes 100%.

### <Summary>

As is clear from the above description, in the material development support system 100 according to the second embodiment, the composition optimization device 130 includes the constraint unit 2301, and avoids the updated material composition information from becoming a negative value and corrects so that the total value becomes 100% (1.0). With this, according to the material development support system 100 of the second embodiment, appropriate material composition information can be searched for while achieving substantially the same effects as those of the first embodiment.

### [Third Embodiment]

In the first and second embodiments described above, the default material composition information is not described in detail, but a case is also assumed where the material composition information having the phase fraction substantially the same as the target phase fraction cannot be searched for, depending on the default material composition information. Therefore, in the third embodiment, the default material composition information is started from the material composition information having a phase fraction relatively similar to the target phase fraction so that the material composition information having the phase fraction substantially the same as the target phase fraction can be searched for. In the following, the third embodiment will be described, focusing on the differences from the first and second embodiments.

### <Functional Configuration of Composition Optimization Device>

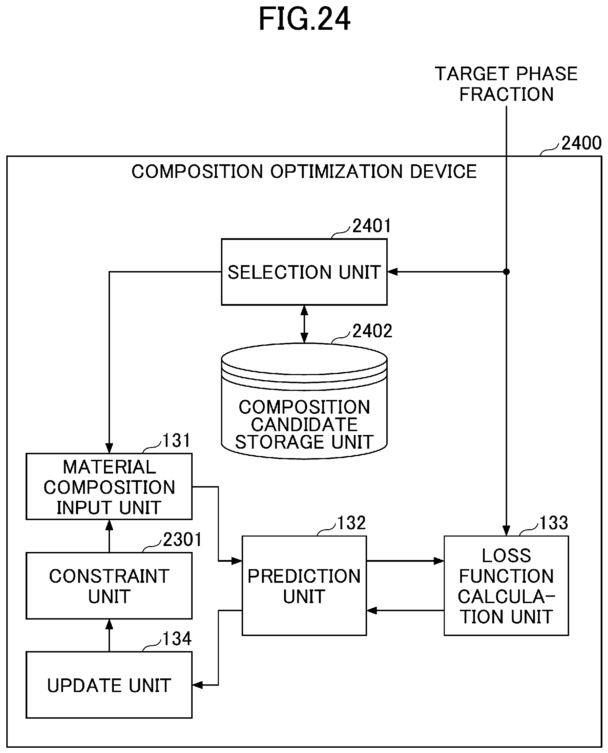

First, a functional configuration of a composition optimization device according to the third embodiment will be described. FIG. 24 is a second diagram illustrating another example of the functional configuration of the composition optimization device. The difference from the functional configuration of the composition optimization device 2300 described with reference to FIG. 23 is that a composition optimization device 2400 according to the third embodiment includes a selection unit 2401.

The selection unit 2401 acquires the input target phase fraction, reads material composition information substantially the same as the acquired target phase fraction from a composition candidate storage unit 2402, and inputs the read material composition information into the material composition input unit 131 as the default material composition information.

### <Default Material Composition Information>

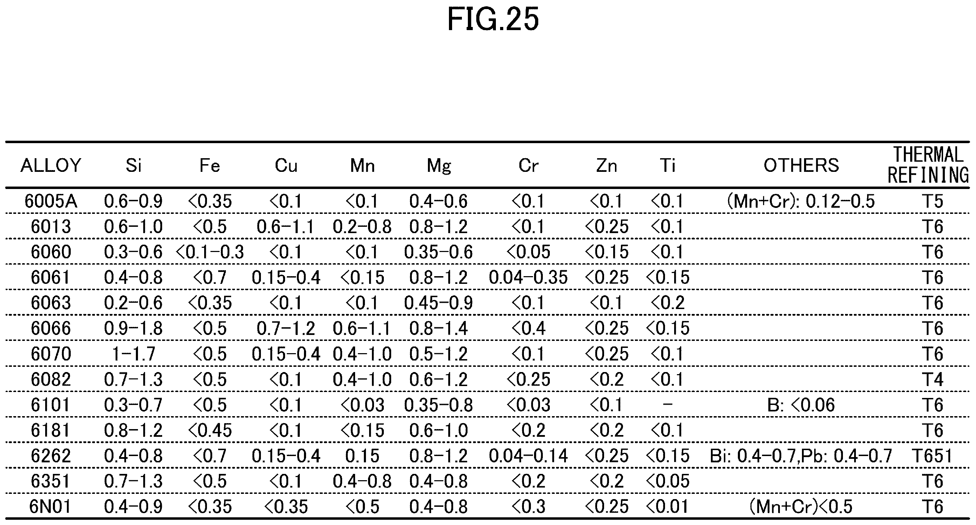

Next, the default material composition information stored in the composition candidate storage unit 2402 will be described. FIG. 25 is a chart indicating an example of the default material composition information. The example of FIG. 25 is an example of the material composition information on the aluminum alloy standard, and indicates the maximum value and the minimum value of the allowable range of the ratio of each composition of 13 types of aluminum alloys.

The selection unit 2401 selects one type of aluminum alloy from among the 13 types of aluminum alloys according to the set target phase fraction. Additionally, the selection unit 2401 sets the minimum value or the maximum value of the allowable range of the ratio of each composition for the selected one type of aluminum alloy as the default material composition information.

### <Processing Result of Composition Optimization Process>

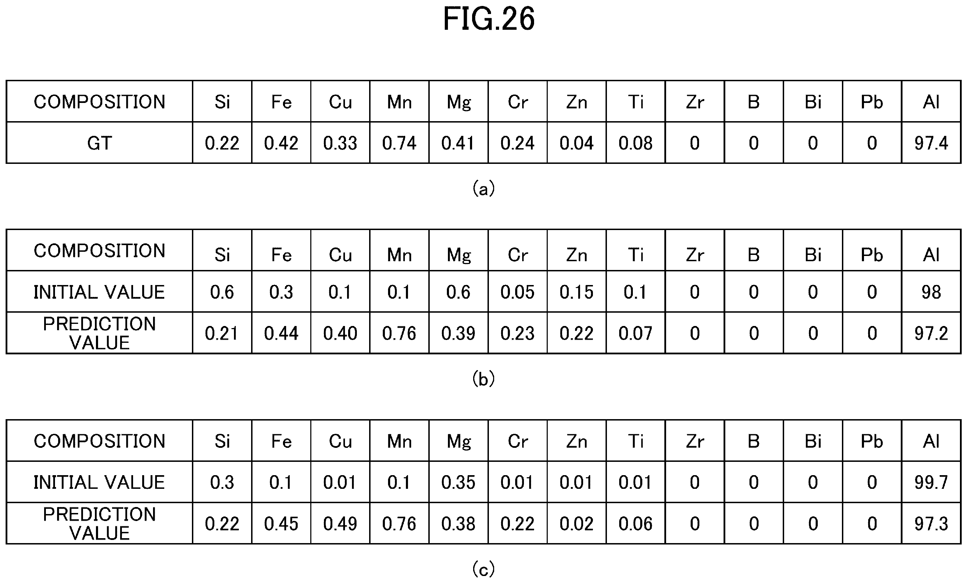

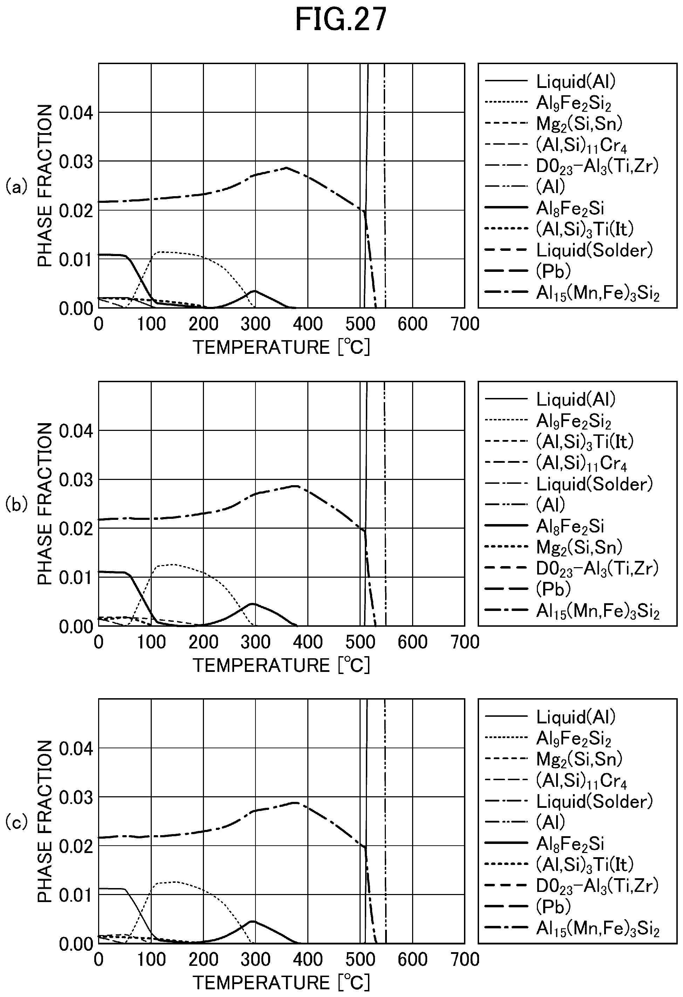

Next, a difference in the processing result of the composition optimization process due to a difference in the default material composition information will be described. FIG. 26 depicts charts indicating examples of default material composition information and the processing result of the composition optimization process. Additionally, FIG. 27 depicts second graphs indicating examples of the processing result of the composition optimization process.

Among these, FIG. 26 (a) indicates material composition information (ground truth data = GT (Ground Truth)) serving as the target phase fraction, and FIG. 27 (a) indicates the respective target phase fractions.

Additionally, FIG. 26 (b) indicates a processing result of the composition optimization process in a case where a predetermined aluminum alloy is selected by the selection unit 2401 as the default material composition information and the maximum value of the allowable range of the ratio of each composition is set as the default material composition information. Additionally, FIG. 27 (b) indicates the phase fractions in this case.

FIG. 26 (c) indicates a processing result of the composition optimization process in a case where a predetermined aluminum alloy is selected by the selection unit 2401 as the default material composition information and the minimum value of the allowable range of the ratio of each composition is set as the default material composition information. Additionally, FIG. 27 (c) indicates the phase fractions in this case.

As is clear from the comparison between FIG. 27 (a) and FIG. 27 (b) and between FIG. 27 (a) and FIG. 27 (c), due to appropriate default material composition information having being selected by the selection unit 2401, a phase fraction substantially the same as the target phase fraction has been obtained in any one of the cases. Additionally, as is clear from the comparison between FIG. 26 (a) and FIG. 26 (b) and between FIG. 26 (a) and FIG. 26 (c), due to appropriate default material composition information having been selected by the selection unit 2401, material composition information substantially the same as the ground truth data can be searched for.

However, as is clear from the comparison between FIG. 26 (a) and FIG. 26 (b), when the maximum value of the allowable range of the ratio of each composition is set as the default material composition information, the error of the composition ratio of "Zn" is great. On contrary, as is clear from the comparison between FIG. 26 (a) and FIG. 26 (c), when the minimum value of the allowable range of the ratio of each composition is set as the default material composition information, the error is small in the ratio of any composition.

The reason why the difference in the composition ratio of "Zn" arises is that, in the case of the aluminum alloy, "Zn" is in a solid solution state, phase deposition does not occur, and "Zn" does not appear in the phase fraction, and therefore, the composition ratio of "Zn" is drawn by the default material composition information. That is, the ratio of the composition of which the phase does not appear in the phase fraction has low reliability, and is a ratio to be finally adjusted by the user. Therefore, when the processing result of the composition optimization process is presented to the user, it may be explicitly indicated to the user that the ratio of the composition of which the phase does not appear in the phase fraction is a ratio to be adjusted by the user.

### <Summary>

As is clear from the above description, the composition optimization device according to the third embodiment selects, as the default material composition information, material composition information having the phase fraction similar to the target phase fraction, and starts the composition optimization process.

With this, according to the composition optimization device of the third embodiment, the material composition information having the phase fraction substantially the same as the target phase fraction can be searched for.

Here, in the above description, a user interface for selecting the material composition information having the phase fraction similar to the target phase fraction has not been described. However, for example, in the case of the aluminum alloy, the phase fraction may be presented to the user with respect to multiple standard alloys (aluminum alloys defined by the standard) having known phase fractions. This allows the user to select a phase fraction similar to the target phase fraction from the multiple presented phase fractions, and select the material composition information on the corresponding standard alloy as the default material composition information.

Alternatively, among the multiple standard alloys having known phase fractions, a standard alloy having a phase fraction similar to the target phase fraction may be presented to the user as a candidate for the default material composition information. Alternatively, an allowable range of the ratio of each element of the standard alloy having the phase fraction similar to the target phase fraction may be presented to the user. This allows the user to set the default material composition information by inputting the ratio of each element within the presented allowable range.

### [Fourth Embodiment]

In the third embodiment described above, the case where the target phase fraction at each temperature within the predetermined temperature range is set as the target phase fraction has been described. However, the method of setting the target phase fraction is not limited thereto, and only the target phase fraction at a specific temperature (here, 100°C) may be set first.

In this case, the selection unit 2401 determines material composition information that achieves the target phase fraction at 100°C, and sets the determined material composition information as the default material composition information. Additionally, in this case, the simulation unit 503 operates the simulator with respect to the set default material composition information, and calculates the phase fraction at each temperature within the predetermined temperature range (here, the phase fractions other than the phase fraction at 100°C).

Then, the phase fraction at each temperature (except for 100°C) within the predetermined temperature range calculated by the simulation unit 503 and the target phase fraction at 100°C that is set first are set as the target phase fraction at each temperature within the predetermined temperature range.

With this, the composition optimization device 2400 can search for the material composition information to have the set target phase fraction at each temperature within the predetermined temperature range based on the set default material composition information.

### [Fifth Embodiment]

In each of the embodiments described above, the description provided as the phase fraction error (logarithmic value) calculation unit 903 comparing the phase fractions (the ground truth data) at the respective temperatures of 100°C to 700°C with the phase fractions (the output data) at the respective temperatures of 100°C to 700°C, and calculating the error of the logarithmic value of the phase fraction at each temperature. However, the method of calculating the error of the logarithmic value by the phase fraction error (logarithmic value) calculation unit 903 is not limited thereto. For example, when the logarithmic value of the phase fraction is calculated, the error may be calculated after the logarithmic value of the phase fraction is made non-negative by adding a value corresponding to the first decimal place of the phase fraction.

Additionally, in each of the above embodiments, the prediction direction in the case where the prediction device 120 "sequentially predicts the phase fraction for each predetermined temperature interval" is not particularly described. However, the prediction direction may be a direction in which the temperature increases or a direction in which the temperature decreases. Alternatively, the prediction direction may be both a direction in which the temperature increases and a direction in which the temperature decreases.

Additionally, in each of the above embodiments, as a specific example of the material composition, the alloy composition indicating the ratio of the additive element (another metal element or a non-metal element) to the metal element contained in the alloy has been described, but the material composition is not limited to the alloy composition. For example, the material composition may be a chemical composition indicating the ratio of each chemical component contained in a substance other than an alloy.

Additionally, in each of the above embodiments, the description is provided as either Seq2Seq with attention mechanism; or Transformer being applied as the trained prediction model. However, the trained prediction model is not limited thereto, and another architecture may be applied as long as the architecture can calculate the time series data, which is data for each predetermined time interval. For example, a recurrent neural network (RNN), a bidirectional RNN, Seq2Seq, or the like may be applied. Alternatively, a gated recurrent unit (GRU), a long short term memory (LSTM), or the like may be applied.

Additionally, in each of the above embodiments, the training device 110 and the prediction device 120 are separately configured, but the training device 110 and the prediction device 120 may be integrally configured. Similarly, in each of the above embodiments, the training device 110 and the composition optimization device 130 are separately configured, but the training device 110 and the composition optimization device 130 may be integrally configured. Similarly, in each of the above embodiments, the prediction device 120 and the composition optimization device 130 are separately configured, but the prediction device 120 and the composition optimization device 130 may be integrally configured.

Additionally, in each of the above embodiments, a usage scene of the search result of the material composition information according to the target phase fraction is not described, but for example, material design and material development may be performed based on the search result of the material composition information according to the target phase fraction.

Here, the present invention is not limited to the configurations described herein, such as the configurations in the above embodiments, combinations with other elements, and the like. These points can be changed without departing from the scope of the present invention, and can be appropriately determined according to the application form thereof.

This application claims priority to Japanese Patent Application No. 2022-166274 filed on October 17, 2022, the entire contents of which are incorporated herein by reference.

### Description of reference symbols

100: material development support system
110: training device
111: training data generation unit
112: training unit
120: prediction device
121: material composition input unit
122: prediction unit
123: display unit
130: composition optimization device
131: material composition input unit
132: prediction unit
133: loss function calculation unit
134: update unit
400: training data
501: element information input unit
502: combination determination unit
503: simulation unit
504: storage control unit
601: encoder
602: decoder
603: loss function calculation unit
901: appearance/disappearance temperature phase fraction error calculation unit
902: phase fraction error calculation unit
903: phase fraction error (logarithmic value) calculation unit
904: phase fraction error (difference value) calculation unit
905: cross entropy error calculation unit
906: weighted addition unit
1101: trained encoder
1102: trained decoder
2300: composition optimization device
2301: constraint unit
2400: composition optimization device
2401: selection unit

## Claims

1. A composition optimization device comprising:
a prediction unit configured to predict, by inputting a predetermined material composition into a trained model, a phase fraction of a material having the predetermined material composition at each temperature within a predetermined temperature range, the trained model being trained using training data in which a material composition of a training target material and a phase fraction of the training target material at each temperature within the predetermined temperature range are associated with each other; and
an update unit configured to update the predetermined material composition input into the trained model by performing backpropagation of an error calculated based on a target phase fraction and the predicted phase fraction,
wherein a process of the prediction unit predicting a phase fraction based on the updated material composition and a process of the update unit updating a predetermined material composition by performing backpropagation of a calculated error are repeated until the calculated error satisfies a predetermined condition.

2. The composition optimization device as claimed in claim 1, wherein the trained model is configured to predict the phase fraction at an (i+1)-th temperature by using phase fractions predicted by the trained model with respect to temperatures up to an i-th temperature (i is an integer of 1 or greater) within the predetermined temperature range.

3. The composition optimization device as claimed in claim 2, wherein an architecture configured to calculate time series data is applied to the trained model, and the trained model predicts the phase fraction for each predetermined temperature interval based on the predetermined material composition, the time series data being data for each predetermined time interval.

4. The composition optimization device as claimed in claim 3, wherein the trained model is any one of an RNN, a bidirectional RNN, Seq2Seq, Seq2Seq with an attention mechanism, a GRU, an LSTM, or Transformer.

5. The composition optimization device as claimed in claim 4, wherein the trained model includes:
an encoder configured to output a feature by the predetermined material composition being input; and
a decoder configured to predict the phase fraction at the (i+1)-th temperature by the output feature and the predicted phase fractions at the temperatures up to the i-th temperature being input.

6. The composition optimization device as claimed in any one of claims 1 to 5, wherein the phase fraction is the phase fraction in a thermodynamic equilibrium state.

7. The composition optimization device as claimed in any one of claims 1 to 6, further comprising a constraint unit configured to correct the updated predetermined material composition so that the updated predetermined material composition that is updated by the update unit satisfies a constraint related to a material composition.

8. The composition optimization device as claimed in claim 7, wherein the constraint unit corrects the updated predetermined material composition so that a total value of the material composition is 100% and a ratio of each composition is 0% or greater.

9. The composition optimization device as claimed in any one of claims 1 to 8, further comprising a loss function calculation unit configured to calculate the error calculated based on the target phase fraction and the predicted phase fraction.

10. The composition optimization device as claimed in claim 9, wherein the error calculated by the loss function calculation unit includes at least:
a first addition result obtained by adding, for all phases, the error between the predicted phase fraction and the target phase fraction at a temperature at which each phase specified based on the target phase fraction appears or disappears;
a second addition result obtained by adding the error between the predicted phase fraction and the target phase fraction at each temperature for the predetermined temperature range;
a third addition result obtained by adding an error between a logarithmic value of the predicted phase fraction and a logarithmic value of the target phase fraction at each temperature for the predetermined temperature range;
a fourth addition result obtained by adding an error between a difference value between phase fractions at adjacent temperatures among the predicted phase fraction at each temperature and a difference value between phase fractions at adjacent temperatures among the predicted phase fraction at each temperature for the predetermined temperature range, or
a fifth addition result obtained by adding an error between a ratio of the predicted phase fraction and a ratio of the target phase fraction at each temperature for the predetermined temperature range.

11. The composition optimization device as claimed in claim 10, wherein the loss function calculation unit performs weighted addition on the first addition result to the fifth addition result.

12. The composition optimization device as claimed in claim 10, wherein the loss function calculation unit makes the logarithmic value of the phase fraction non-negative by adding a value corresponding to a first decimal place of the phase fraction when calculating the logarithmic value of the phase fraction.

13. The composition optimization device as claimed in any one of claims 1 to 12, wherein the predetermined material composition is a material composition having a phase fraction similar to the target phase fraction selected from material compositions defined by a standard.

14. A composition optimization method performed by a computer of a composition optimization device, comprising:
a prediction step of predicting, by inputting a predetermined material composition into a trained model, a phase fraction of a material having the predetermined material composition at each temperature within a predetermined temperature range, the trained model being trained using training data in which a material composition of a training target material and a phase fraction of the training target material at each temperature within the predetermined temperature range are associated with each other; and
an update step of updating the predetermined material composition input into the trained model by performing backpropagation of an error calculated based on a target phase fraction and the predicted phase fraction,
wherein a process of predicting a phase fraction based on the updated material composition in the prediction step and a process of updating a predetermined material composition by performing backpropagation of a calculated error in the update step are repeated until a calculated error satisfies a predetermined condition.

15. A composition optimization program for causing a computer of a composition optimization device to perform steps comprising:
a prediction step of predicting, by inputting a predetermined material composition into a trained model, a phase fraction of a material having the predetermined material composition at each temperature within a predetermined temperature range, the trained model being trained using training data in which a material composition of a training target material and a phase fraction of the training target material at each temperature within the predetermined temperature range are associated with each other; and
an update step of updating the predetermined material composition input into the trained model by performing backpropagation of an error calculated based on a target phase fraction and the predicted phase fraction,
wherein a process of predicting a phase fraction based on the updated material composition in the prediction step and a process of updating a predetermined material composition by performing backpropagation of a calculated error in the update step are repeated until a calculated error satisfies a predetermined condition.
